# EUROPEAN PATENT SPECIFICATION

(11) **EP 0 668 912 B1**
(45) Date of publication and mention of the grant of the patent: **15.12.1999**
(21) Application number: 93925072.6
(22) Date of filing: 26.10.1993
(51) Int. Cl.: C12N 15/12, C07K 14/705, C12P 21/08, C12N 5/10, G01N 33/50

(54) **THE St-B17 SEROTONIN RECEPTOR**
ST-B17 SEROTONIN REZEPTOR
RECEPTEUR DE SEROTONINE St-B17

(30) Priority: 26.10.1992 US 970338
(43) Date of publication of application: 30.08.1995
(73) Proprietor: THE GOVERNMENT OF THE UNITED STATES OF AMERICA, as represented by THE SECRETARY OF THE DEPARTMENT OF HEALTH AND HUMAN SERVICES, Rockville, MD 20852-3804 (US)
(72) Inventor: SIBLEY, David, R., Rockville, MD 20855 (US); MONSMA, Frederick, J., Jr., Baltimore, MD 21227 (US); HAMBLIN, Mark, W., Seattle, WA 98115 (US)
(74) Representative: VOSSIUS & PARTNER
(86) International application number: PCT/US93/10296
(87) International publication number: WO 94/10310

(56) References cited:
- WO-A-91/17174
- MOLECULAR PHARMACOLOGY vol. 43, no. 3 , March 1993 , BALTIMORE (MD) , USA pages 320 - 327 MONSMA, F.J.JR. ET AL.; 'Cloning and expression of a novel serotonin receptor with high affinity for tricyclic psychotropic drugs'
- DATABASE WPI Week 9319, Derwent Publications Ltd., London, GB; AN 93-159491 'Cloned gene encoding serotonin St-B17 receptor gene - used to test drugs for CNS activity' & US,A,7 970 338 (US DEPT HEALTH AND HUMAN SERVICE) 1 April 1993
- TRENDS IN PHARMACOLOGICAL SCIENCES vol. 13 , 1992 , CAMBRIDGE GB pages 160 - 165 HEN, R. ; 'Of mice and flies : Commonalities among 5-HT receptors'

## Description

### Scope of the invention

This invention relates to cloning and characterization of cellular receptors. Specifically, this invention relates to the cloning and characterization of the St-B17 serotonin receptor protein.

### Background

The neurotransmitter serotonin (5-hydroxytryptamine, 5-HT) has a variety of functions in the central nervous system. It has been implicated in many cognitive and behavioral functions, including aggression, sexual behavior, learning and sleep. Disruptions of serotonergic systems may be a critical factor in a number of clinical disorders or conditions including schizophrenia, depression, obsessive compulsive disorder, anxiety, migraine headaches, and pain.

The multitude of effects produced by serotonin are mediated by various serotonin receptors which exist in the central and peripheral nervous system. Pharmacological studies as well as molecular cloning of 5-HT receptors have revealed a multiplicity of receptor subtypes with very different affinities for 5-HT and different physiological function (Hen, TiPS 13, 160-165 (1992)). The transduction of serotonergic signals across the neuronal membrane is mediated by a diversity of receptor subtypes which, in mammals, appear to fall into four pharmaceutically distinct classes designated 5-HT₁ - 5-HT₄. The 5-HT₁ subcategory has been further subdivided into five different subtypes referred to as 5-HT_{1A-E}. The primary structures for a number of these receptors have been elucidated by molecular cloning, including the 5-HT₁, 5-HT₂ and 5HT₃ subclasses. In addition, the sequences of three different Drosophila serotonin receptors, 5-HT_{dro1} and 5-HT_{dro2A,B}, have been reported. (For review see Hen, Tips 13, 160-165 (1992)).

Selective therapeutic agents, including agonist and antagonist drugs, have been developed based on serotonin receptor technologies. 5-HT₂ antagonists, for example, are useful in the treatment of schizophrenia, parkinsonism, and anxiety disorders. Several azapirones, such as buspirone, gepirone, and ipsapirone, have high affinities for 5-HT_{1A} receptors in the brain, and are useful in the treatments of anxiety. Highly selective 5-HT uptake inhibitors, which have minimal effects on norepinephrine or dopamine uptake or on other neurotransmitter receptors, have been used to successfully treat depression.

Characterization of all specific 5-HT receptors would clarify the role of serotonin in the central nervous system. Analysis of the receptor subtypes and their functional role in the central nervous system would help elucidate the pathophysiological basis of many human diseases. Accordingly, there is a need for identification and characterization of all specific 5-HT receptors, and the development of selective therapeutic agents based on these receptor technologies.

### Description of the Figures

**Figure 1**: Saturation analysis of specified [¹²⁵I]-LSD binding to membranes prepared from Cos-7 cells transiently transfected with construct pSRα-B17. The experiment shown is representative of 3 independent experiments conducted in triplicate. Inset, scatchard analysis of saturation binding data. In this experiment the K_{D} and Bₘₐₓ values were 1.5 nM and 3.4 pMoles mg protein⁻¹, respectively.

**Figure 2**: Pharmacological analysis of [¹²⁵I]-LSD binding to Cos-7 cell membranes expressing clone St-B17. Competition curves shown are representative of 3 independent experiments conducted in triplicate.

**Figure 3**: Northern blot analysis of St-B17 RNA transcripts in rat brain. Each lane contained 3 µg of poly (A+) RNA. Lanes: AMYG, amygdala; CB, cerebellum; CTX, cerebral cortex; HIP, hippocampus; HYP, hypothalamus; MED, medulla; OB, olfactory bulb; OT, olfactory tubercle; PIT, pituitary; RET, retina; STR, striatum; THAL, thalamus. Locations of RNA size (kb) markers are indicated.

**Figure 4**: Accumulation of cAMP in response to agonist activation of St-B17 stably expressed in HEK-293 cells. This figure shows a dose-response curve activation of St-B17 by serotonin in stably transfected, intact HEK-293 cells.

**Figure 5**: Pharmacological analysis of serotonin stimulated cAMP accumulation in stably transfected HEK-293 cells. DE is an acronym for dihydroergocryptine. All drugs were assayed at 10 µM final concentration.

One embodiment of the present invention is directed at the polynucleotide sequence encoding a mammalian receptor protein St-B17, said protein exhibiting high affinity binding for clozapine, loxapine, and amoxipine and exhibiting high expression levels in limbic and cortical regions of the brain, said nucleotide sequence being selected from a nucleotide sequence comprising SEQ ID NO:7; SEQ ID NO:12; a nucleotide sequence hybridizing under moderate stringency conditions at 6xSSC and 55°C, pH7, to a 1192 bp XmaI-BstXI or a 655 bp BamHI-EagI fragment from Sequence ID NO:7; or a nucleotide sequence encoding a protein having the amino acid sequence of SEQ ID NO:8 or SEQ ID NO:13. Preferably this receptor protein encoded by said nucleotide sequence is human St-B17.

A further embodiment of the present invention includes a recombinant construct of the polynucleotide, preferably the polynucleotide of SEQ ID NO:7, encoding the St-B17 serotonin receptor protein operably linked to a heterologous promoter.

Additionally the present invention encompasses an isolated nucleotide sequence having at least 30 contiguous nucleotides of the St-B17 gene.

The present invention also embodies a mammalian cell line, preferably human, comprising the polynucleotide of the invention, in continuous culture expressing the St-B17 serotonin receptor protein. Particularly, the cell line contains the polynucleotide Sequence ID NO:7. One especially preferred embodiment includes HEK 293 cells in continuous culture expressing the St-B17 receptor.

Another embodiment of the present invention is directed to the St-B17 serotonin protein encoded by any one of the above described nucleotide sequences or produced by a cell line of the present invention.

Yet another embodiment of the present invention includes isolated antibodies against the St-B17 serotonin receptor protein. Preferably these isolated antibodies are polyclonal, even more preferably, the isolated antibodies are monoclonal.

Another embodiment of the present invention is a method for screening a drug candidate for central nervous system activity by contacting the drug candidate with the St-B17 protein and measuring binding of the drug candidate by the protein.

An additional embodiment of the present invention is a method for screening a drug candidate for central nervous system activity by first contacting the St-B17 serotonin receptor protein with a first molecule known to be bound by the protein to form a first complex of the protein and the first molecule; then contacting the first complex with the drug candidate; and finally measuring whether the drug candidate displaces the first molecule from the first complex. This method can preferably include the measuring step, the step of measuring the formation of a second complex of the protein and the drug candidate. An alternative method of measuring the displacement of the first molecule can be performed by measuring the amount of the first molecule that is not bound to the protein.

An even further embodiment of the present invention includes a method of screening a ligand for binding to the St-B17 serotonin receptor by the steps of:
transfecting a cell line with a gene encoding the St-B17 serotonin receptor protein in an expression vector;
culturing the cell line to express the gene in media containing a ligand of the receptor; and
measuring the binding of the ligand to the receptor produced by the cell line.

Preferably, in this method, the cell line is from a mammal, more preferably a human, and most preferably the cell line is HEK 293. An additional preferred embodiment of the above method entails transfecting cells with the St-B17 serotonin receptor encoded by the polynucleotide of SEQ ID NO:7. Additional preferred embodiments of this method include using eukaryotic expression vectors, preferably pSRα-B17 described in example 4 hereinafter. Another preferred embodiment includes using ligands that are labelled and the label is either radioactive or colorimetric.

Still another embodiment of the present invention is a method of determining the ability of a drug to inhibit ligand binding to the St-B17 serotonin receptor protein by the following steps:
transfecting a cell line with a DNA sequence encoding St-B17 in an expression vector;
culturing the cell line to express the St-B17 receptor in media containing a ligand of the receptor;
determining the level of binding of the ligand to the receptor;
culturing the same cell line to express the receptor in the presence of both the ligand and the drug; and
determining the level of binding of the ligand to the expressed receptor, wherein a lower level of binding in the presence of the drug indicates that the compound is an inhibitor of ligand binding.

Preferably, the cell line of this method is mammalian, most preferably HEK 293, and the ligand, the drug or both are labeled. Additionally, the expression vector of this method can preferably be pSRa-B17 and the ligand can advantageously be serotonin.

Yet another embodiment of the present invention is a method for determining the ability of a drug to inhibit ligand binding to the St-B17 serotonin receptor by the following steps:
transfecting a cell line with a gene encoding the St-B17 receptor protein in an expression vector;
culturing the cell line to express the receptor;
determining the level of binding of the ligand to the receptor;
culturing the same cell line to express the receptor in the presence of both the ligand and the drug being tested for inhibition; and
determining the level of cAMP accumulation in the cell, wherein n a lower level of cAMP accumulation in the cell in the presence of the drug indicates inhibition of ligand binding.

Still another embodiment of the present invention is a method of determining the ability of a drug to inhibit ligand binding to the St-B17 serotonin receptor protein by the steps of:
transfecting a cell line with a DNA sequence encoding St-B17 in an expression vector;
culturing the cell line to express the St-B17 receptor in media containing a ligand of the receptor;
isolating the membranes from the cell line, wherein the membranes contain the expressed St-B17 receptor;
determining the level of binding of the ligand to the receptor on the membranes;
incubating the membranes in the presence of both the ligand and the drug; and
determining the level of binding of the ligand to the membranes, wherein a lower level of binding in the presence of the drug indicates that the compound is an inhibitor of ligand binding.

Still another embodiment of the present invention is a method for detecting expression of the St-B17 serotonin receptor gene in a tissue sample by the steps of:
removing a tissue sample from a mammal;
contacting the mRNA in the cytoplasm of the tissue sample with a labeled polynucleotide probe having homology to the St-B17 serotonin receptor gene; and
detecting the binding of the probe to the tissue wherein positive binding indicates expression of the St-B17 serotonin receptor gene.

### Detailed Description

We have cloned a new serotonin 5-HT (St-B17) receptor gene from rat and human brain cells. This receptor has a nucleotide sequence that is distinct from any previous gene in the serotonin family. The rat St-B17 gene has a 1311 bp open reading frame coding for a protein with 437 amino acids. The human St-B17 gene has a 1320 bp open reading frame coding for a protein with 440 amino acids.

In addition, this receptor protein exhibits a distinct pharmacological profile not previously described. The unique pharmacology together with the relatively low level of homology (<50%) of St-B17 with previously cloned 5-HT receptor subtypes indicates that this receptor does not belong to any of the previously defined 5-HT₁ - 5-HT₄ subcategories of 5-HT receptors. Thus, St-B17 represents an unknown and uncharacterized receptor.

The molecular cloning of St-B17 will be useful for developing drugs which interact with 5-HT receptors. For instance, the cloning of this novel gene allowed us to develop new assays for discovering serotonin receptor binding and inhibitory drugs. These assays were performed by transfecting cells, such as Cos-7 mammalian cells, with the gene encoding the St-B17 serotonin receptor protein in an expression plasmid. The Cos-7 cells were then placed in media and expressed the St-B17 receptor on their cell surface. Radiolabeled ligands were then tested for binding to these transfected cells.

By using this method we were able to screen many drugs for their binding activity to the serotonin receptor. Similarly, this assay system was used to detect competitive inhibitors of serotonin binding. By incubating the transfected cells in the presence of a radiolabeled known binding molecule (LSD) we were able to test various drugs for their ability to inhibit this binding. Table 1 below describes a suite of drugs that had wide-ranging affects on ¹²⁵I-LSD binding to the rat St-B17 serotonin receptor.

Since serotonin binding is intimately involved in the functionality of the central nervous system, assays for detecting drugs which bind or block serotonin receptors are of paramount importance. As discussed in the background, many behavioral functions are believed to be mediated through serotonergic systems. For researchers to be able to accurately assess the projected effects of a new serotonin-related drug in humans, it is important to test that drug's effect on every known serotonin receptor. The discovery of the St-B17 receptor provides the researcher with a previously unknown assay to study possible *in vivo* effects of the drug being tested.

It is also anticipated that isolated St-B17 receptor protein will be useful for performing assays to determine agents which bind or inhibit binding to serotonin receptors. In discussing the isolated St-B17 receptor protein, we are referring to not only affinity-purified protein, but also receptor proteins bound in a membrane and removed from the cell. For example, we transfected Cos-7 cells with the gene encoding the St-B17 receptor protein in an expression plasmid. After incubation in media to allow St-B17 receptor expression the cells were lysed and the membranes (containing the bound St-B17 receptors) were pelleted. These membranes, isolated from the host cells were used for radiolabeled ligand binding studies.

The present invention includes isolated St-B17 serotonin receptors from rats, humans, other mammals, and other vertebrates. Further investigations of St-B17's role in vivo and *in vitro* will help improve current therapies for several neuropsychiatric disorders by providing additional information on each drug's receptor-mediated action.

For the first step in cloning the St-B17 receptor we used the polymerase chain reaction (PCR) to selectively amplify cDNA sequences from mRNA purified from rat corpus striatum. Poly(A)⁺ RNA from the rat striatum was used to first synthesize a cDNA library by reverse transcription using methods well known in the art. The following example shows one method for amplifying the serotonin receptor genes using PCR amplification.

### Example 1: PCR Amplification of Rat Striatum mRNA

We used a pair of highly degenerate primers, with sequences which were derived from the third and sixth transmembrane (TM) regions of previously cloned G protein-linked receptors in multiple rounds of PCR to amplify the serotonin gene sequences in the striatum library.

Polymerase chain reaction amplification was carried out as previously described (Monsma, F.J., Jr., Mahan, L.C., McVittie, L.D., Gerfen, C.R. & Sibley, D.R., Proc. Natl. Acad. Sci. USA **87**, 6723-7 (1990)) using cDNA prepared from rat striatal mRNA and 1 µM each of the following primers:

The PCR timing cycle was 1.5 min at 93°C, 2 min at 50°C, and 4 min at 72°C followed by a 7 min extension at 72°C. The reaction products were purified and size fractionated on a 1% agarose gel. Individual bands were excised, electroeluted, concentrated by ultrafiltration, and ligated into the pCR1000© vector (INVITROGEN). Competent INVaF bacterial cells (INVITROGEN) were transformed and mini-preparations of plasmid DNA prepared for insert sequencing.

This process resulted in the amplification of several cDNA fragments ranging from 300 to approximately 500 base pairs (bp) in size. Each fragment was preliminarily characterized by DNA sequence analysis. Several of these cDNA fragments exhibited sequence homology with previously cloned G-protein coupled receptors such as serotonin, one of which, clone St-B17₁ (538 bp), was particularly homologous with several catecholamine receptors and was thus selected for further characterization.

We selected 1 X 10⁶ recombinants from the rat striatal cDNA library, constructed in the λ ZAP II© vector (STRATAGENE, La Jolla, CA), to be screened with the 538 bp St-B17₁ PCR fragment. The 538 bp fragment was made into a probe by ³²P-labeling (via nick translation) using well known methods. Duplicate nitrocellulose filters were hybridized in 50% formamide, 0.75 M NaCl, 0.075 M sodium citrate (5X SSC), 5X Denhardt's solution 0.02 M Na₂HPO₄, 0.25% SDS, 0.15 mg/ml salmon sperm DNA, and 4 x 10⁶ dpm/ml of ³²P-labeled probe for 24 hr at 37°C. High stringency washing of the filters was performed with 1X SSC and 0.1% SDS at 65°C prior to autoradiography.

The λ phage found to hybridize with the probe were subsequently plaque purified. *In vivo* excision and rescue of the nested pBluescript plasmids from the λ ZAP II clones was performed using helper phage according to the STRATAGENE protocol.

Briefly, the λ phages carry a nested pBluescript plasmid which can be excised via the STRATAGENE protocol. This nested plasmid carried the St-B17 gene and could be used for subsequent sequencing and transfection. Nucleotide sequence analysis was performed using the Sanger dideoxy nucleotide chain termination method with Sequenase© (US BIOCHEMICAL CORP.) on the excised denatured double-stranded plasmid templates.

One positive clone (St-B17₂) from the above screening, containing a cDNA insert of 2.8kb, was isolated and nucleotide sequenced. The largest open reading frame was 879 bp long encoding a protein containing sequences homologous to transmembrane (TM) regions I-VI of previously cloned biogenic amine receptors.

Further analysis of the 2.8kb cDNA revealed the presence of a sequence 210 bp beyond the end of the open reading frame which was homologous to the seventh transmembrane (TM) region of several receptors. Careful examination of the DNA sequence between these putative TM regions VI and VII revealed the presence of consensus donor and acceptor sequences for intron/exon splice junctions suggesting the possibility than this region represented an un-spliced intron having an inframe stop codon. Sequence ID NO: 9 is a partial polynucleotide sequence of the 2.8kb clone having the entire coding sequence, and the internal intron.

This would explain the homology to the seventh TM region in the purported non-coding portion of the gene. The possibility that we had isolated a cDNA from a partially spliced mRNA was examined, as described in Example 2 below, using PCR to amplify this putative intron region from cDNA prepared from rat striatal mRNA.

### Example 2: Amplification and Analysis of the 2.8kb Insert

Primers P3 and P4 were synthesized to have homology near to the 3' and 5' ends of the putative splice region. The sequence of primer P4 is complementary to the coding sequence since it was designed to anneal with the homologous DNA strand. With these primers, we would either isolate 223 bp sequences corresponding to a spliced message, or 416 bp sequences corresponding to an unspliced message. This system could thereby indicate if the putative intron acceptor/donor sites were really utilized for splicing reactions in the cell.

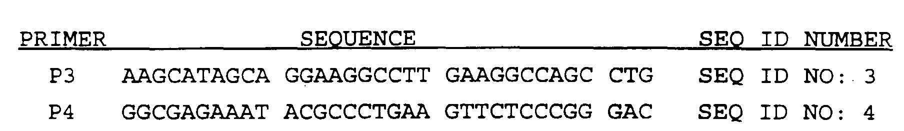

Amplification across the putative splice region with primers P3 and P4 resulted in a primary DNA product of 223 bp, the size predicted for mRNA spliced at the observed donor/acceptor sites, and a minor band of 416 bp, the size expected based on the sequence of the unspliced St-B17₂ cDNA clone. We thus believed that the St-B17₂ cDNA clone was derived from a minor, incompletely spliced mRNA.

Since an additional screen of the cDNA library did not result in the isolation of a full-length, completely spliced clone, we used PCR following the protocol outlined in Example 1 to amplify the predicted coding sequence from rat striatal mRNA.

### Example 3: Amplification of Putative Coding Region

We used primers P1 and P2 (shown below as SEQ ID NOS: 5 and 6, respectively) and PCR to amplify the 2.8kb clone isolated in Example 2 from the rat striatum cDNA library. Primer P2 was complementary to the coding sequence since it was designed to hybridize with the homologous nucleotide strand.

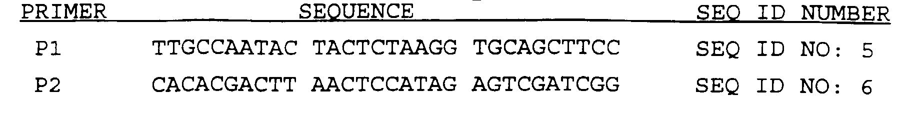

Two nucleotide sequences of 1.6 and 1.4 kb-were obtained from the PCR reaction, the latter of which (St-B17₃)was subcloned into plasmid pBluescript II© SK (+). Sequence analysis confirmed the identity of St-B17₃ and revealed that the putative intron was absent in this clone (SEQ ID NO: 7) with the flanking exons spliced together at nucleotide 873 as predicted. Splicing at this position resulted in a 1,311 bp open reading frame encoding a protein of 437 amino acid residues (SEQ ID NO: 8) with a calculated molecular weight of 46.8 kD.

Hydropathy analysis of the deduced amino-acid sequence of the spliced St-B17₃ protein indicated seven hydrophobic regions predicted to represent putative transmembrane spanning domains. When compared to previously cloned G-protein coupled receptors, the transmembrane regions of St-B17 exhibited high homology to various serotonin (5-HT) receptors suggesting that it may be a member of this receptor family.

Within the transmembrane regions, St-B17₃ exhibited homologies of 41%, 40%, 39%, 38%, 37% and 36% with 5-HT₂, 5-HT_{1D}, 5-HT_{1C}, 5-HT_{1B}, 5-HT_{1A} and 5-HT_{1E} receptors, respectively. The third cytoplasmic loop of St-B17₃, consisting of 57 residues, would be the shortest of the cloned 5-HT receptors, though similar in length to the 5-HT_{1C} and 5-HT₂ receptors. These receptors have been described previously by Julius, D. et al., Science **241**, 558-564 (1988); and Julius, D. et al., Proc. Natl. Acad. Sci. USA **87**, 928-932 (1990); and Pritchett, D.B., et al., EMBO J. **7**, 4135-4140 (1988). Similarly, while the 117 residue carboxy-terminal tail of St-B17₃ is the longest amongst the 5-HT receptors, it is similar in length to that found in the 5-HT_{1C} and 5-HT₂ receptors.

The combination of a relatively short third cytoplasmic loop and a long carboxy-terminus is common among receptors which are coupled to the stimulation of either the adenylyl cyclase or phospholipase C signal transduction systems. St-B17₃ also contained one potential N-linked glycosylation site at Asn-9 in the extracellular amino terminus. Additionally, several potential sites for phosphorylation by the cyclic AMP-dependent protein kinase or protein kinase C in both the third cytoplasmic loop and the intracellular carboxy-terminal tail were deduced.

To definitively establish the identity of the receptor encoded by St-B17₃, we wanted to express this gene in mammalian cells. Unfortunately, the PCR-amplified full-length, correctly spliced St-B17₃ cDNA could not be used directly for expression due to several PCR-generated base substitutions in the 5' region up-stream of the internal Bam H1 site.

We thus constructed a hybrid cDNA in which the entire 3'. sequence down-stream of the Bam H1 site from the intron-containing clone (St-B17₂) was replaced with the corresponding Bam H1 fragment from the correctly spliced, PCR-amplified cDNA (St-B17₃) as explained below in Example 4.

### Example 4: Construction of Expression Clone

We cleaved the 1.4kb St-B17₃ clone isolated in Example 3 with BamHI by well known methods. We also cleaved the 2.8kb St-B17₂ clone isolated in Example 1 with BamHI. By mixing and annealing the fragments from these two digestions, we derived a clone (St-B17) with the 5' end of St-B17₂ and the 3' end of St-B17₃. The resultant fusion gene had a functional 5'leader and initiation codon, and a correctly spliced intron/exon junction.

This construct was subcloned into the eucaryotic expression vector pCD-SRα yielding pSRα-B17. This clone was then used to transfect mammalian cells to study the expression, binding, and regulation of the new serotonin receptor gene as discussed below in Example 5.

### Example 5: Transfection of Mammalian Cells with St-B17

Transient transfection of Cos-7 cells with pSRα-B17 resulted in the appearance of high affinity and saturable binding sites for the serotonergic ligand [¹²⁵I]-LSD (lysergic acid diethylamide).

Cos-7 cells were transfected with the pSRα-B17 construct using the calcium phosphate precipitation method as previously described by Monsma, F.J. et al., Proc. Natl. Acad. Sci. USA **87**, 6723-7 (1990). Cells were harvested 72 hours after transfection and either disrupted in a dounce homogenizer in 50 mM Tris-HCl, pH 7.4 at 37°C, 10 mM MgSO₄ and 0.5 m M EDTA, or frozen in 5 mM Tris-HCl, pH 7.4 at 25°C, 5 mM MgCl₂, 250 mM sucrose and stored in liquid N₂ prior to membrane preparation. Crude membranes were prepared from cell homogenates by centrifugation at 43,000 x g, and re-suspension in homogenization buffer at a protein concentration of 60 µg/ml.

Once the crude membranes had been prepared from the cell homogenates, we proceeded to pharmacologically screen various drugs for their effect on [¹²⁵I]-LSD to St-B17 as discussed below in Example 6.

### Example 6: Pharmacological Screening Assays Using St-B17

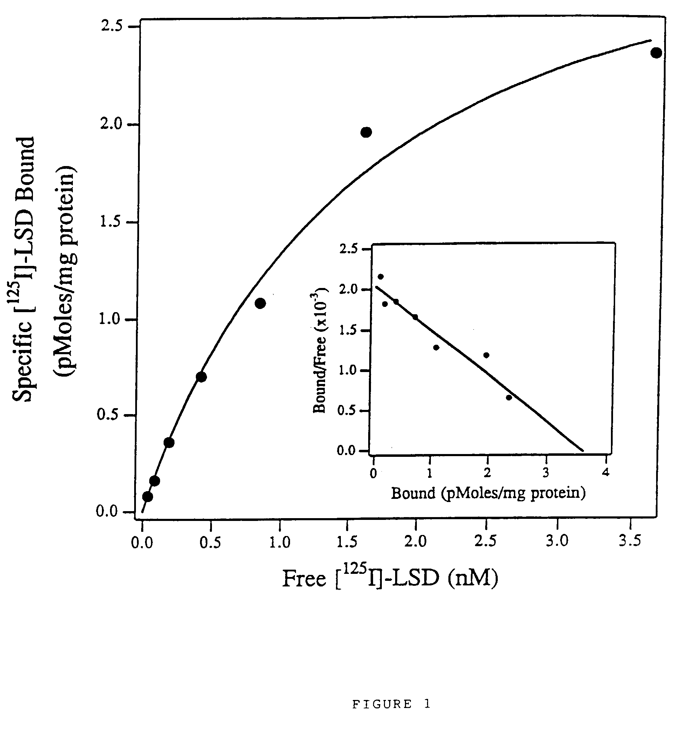

It was first necessary to determine the binding strength and saturation point of LSD, so that we could later analyze drugs that displaced this chemical. Radiolabeled LSD ([¹²⁵I]-LSD) exhibited a dissociation constant (K_{D}) of 1.26 (±0.17, n=3) nM and maximum binding (Bₘₐₓ) values ranging from 2-5 pMoles/mg protein (Figure 1) when added to the membranes prepared by the method of Example 5. Figure 1 shows the saturation analysis of [¹²⁵I]-LSD binding to membranes prepared from Cos-7 cells transiently transfected with construct pSRα-B17. The experiment reported is representative of 3 independent experiments conducted in triplicate. The inset (Figure 1) is a scatchard analysis of the saturation binding data. In this experiment the K_{D} and Bₘₐₓ values were 1.5 nM and 3.4 pMoles mg protein⁻¹, respectively. This assay shows the strong binding of LSD to the St-B17 receptor.

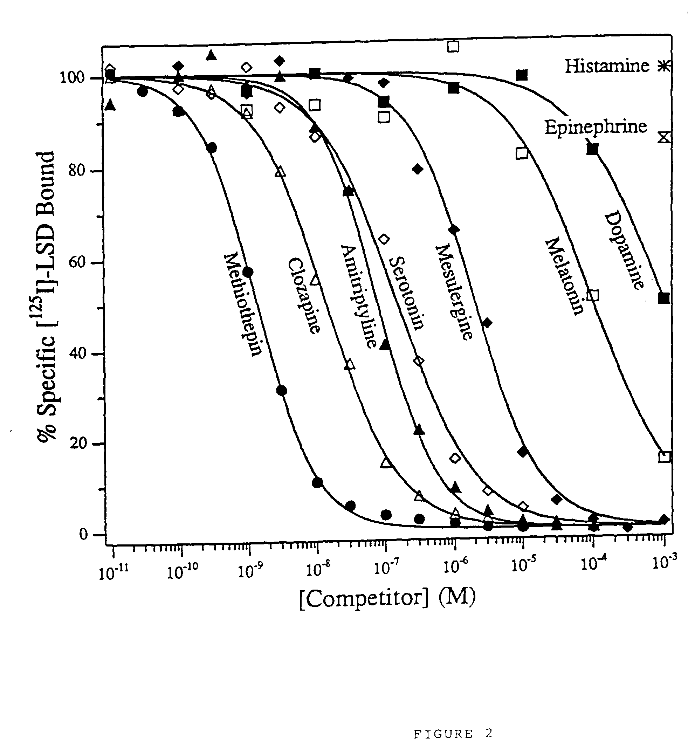

Figure 2 displays the results of a pharmacological competition assay to analyze the displacement of [¹²⁵I]-LSD in the presence of various serotonin receptor binding drugs. This experiment was performed on Cos-7 cell membranes expressing the St-B17 serotonin receptor. The competition curves shown are representative of 3 independent experiments conducted in triplicate. Average inhibition constants (Kᵢ) and SEM values are given in Table 1. No specific binding of [¹²⁵I]-LSD was observed in untransfected Cos-7 cells or in cells transfected with the pCD-SRα expression vector alone (controls).

**Table 1.**

| Pharmacology of St-B17 Expressed in Cos-7 cells | | |
|---|---|---|
| Drug | K₁ (nM) Mean ± SEM | |
| | vs. [¹²⁵I]-LSD | vs. [³H]-5-HT |
| Methiothepin | 1.84 ± 0.23 | 0.39 ± 0.063 |
| Lisuride | 8.19 ± 0.48 | 5.3 ± 1.3 |
| Clozapine | 12.87 ± 1.69 | 20 ± 2.5 |
| Dihydroergotamine | 13.07 ± 0.90 | 5.4 ± 5.4 |
| 2-Br-LSD | 17.14 ± 1.11 | |
| Pergolide | 29.87 ± 7.31 | |
| Metergoline | 29.97 ± 0.38 | 61 ± 23 |
| Amoxipine | 30.40 ± 1.64 | |
| Lergotrile | 36.45 ± 1.04 | |
| 5-methoxytryptamine | 38.92 ± 3.95 | 18 ± 2 |
| Ritanerin | 44.12 ± 4.35 | 16 ± 2 |
| Mianserin | 45.74 ± 8.93 | 38 ± 7 |
| Clomiprimine | 53.78 ± 3.28 | |
| 5-Hydroxy-N,ω-methyltryptamine | 57.95 ± 8.32 | |
| Loxapine | 64.75 ± 0.60 | 56 ± 1.5 |
| amitriptyline | 69.64 ± 7.21 | 82 ± 6 |
| N,N-Dimethyl-5-methoxytryptamine | 79.81 ± 3.86 | |
| 1(1-naphthyl)piperazine | 103.61 ± 13.81 | |
| 5-Benzyloxytryptamine | 110.18 ± 17.90 | |
| Cyproheptadine | 133.85 ± 6.17 | |
| Doxepin | 135.74 ± 7.11 | |
| Nortriptyline | 147.49 ± 2.61 | |
| 5-HT | 151.01 ± 12.78 | 56 ± 9 |
| Dihydroergocryptine | 160.70 ± 13.21 | |
| Imipramine | 208.49 ± 22.76 | 190 ± 3 |
| N,ω-methyltryptamine | 341.60 ± 32.21 | |
| Methysergide | 371.74 ± 72.66 | |
| Tryptamine | 438.27 ± 14.84 | |
| TFMPP | 482.37 ± 37.21 | |
| CGS 12066B | 737.69 ± 66.63 | |
| 5-Carboxamidotryptamine | 774.35 ± 84.39 | 253 ± 20 |
| PAPP | 805.62 ± 75.17 | |
| Mesulergine | 1718.88 ± 248.35 | |
| Fluoxetine | 1765.36 ± 290.37 | |
| mCPP | 2309.60 ± 343.05 | |
| 2-MPP | 3054.95 ± 321.06 | |

Increasing concentrations of the indicated compounds were used to inhibit the binding of either 0.5 nM [¹²⁵I]-LSD or 5 nM [³H]-5-HT to membranes of Cos-7 cells transiently transfected with clone St-B17. [¹²⁵I]-LSD competition assays were performed by incubating the transfected Cos-7 cells with [¹²⁵I]-LSD and the indicated concentrations of each drug as described above and in Figure 2, while those for [³H]-5-HT were performed as previously described (Hamblin, M.W. & Metcalf, M.A., Mol. Pharmacol. **40**, 143-148 (1991)). Kᵢ values were obtained from graphically determined IC₅₀ values by the method of Cheng and Prussoff (Biochem. Pharmacol. **22**, 3099-3108 (1973)) and are presented as the geometric mean ± SEM (n = 3) calculated according to De Lean et al. (Mol. Pharmacol. **21**, 5-16 (1982)).

This method thereby provides a way of screening drug candidates for central nervous system activity by testing its binding to isolated St-B17. We believe that drugs which bind to the St-B17- receptor *in vitro* will also have effects *in vivo*. In addition to directly measuring the binding of a drug to the St-B17 receptor, we were also able to measure the displacement of known binding ligands to the St-B17 receptor. By measuring the binding of a ligand as discussed above, and then comparing that binding to the level of binding in the presence of a drug candidate, we were able to estimate the potential of the drug to displace the ligand.

The following compounds were screened and found to exhibit inhibition constant (Kᵢ) values >10µM: zimelidine, zacopride, NAN-190, citalopram, BIMU 1, metoclopromide, fenfluramine, MDL 72222, 8-OH-DPAT, BRL 24924, BRL 43694A, GR 38032F, ICS 205,930, ketanserin, melatonin, spiroperidol, tyramine, DAU 6215, DAU 6285, dopamine, epinephrine, histamine, idazoxan, LY 278,584, maxindol, norepinephrine, octopamine, paroxetine, and pindolol.

Preliminary characterization of the St-B17 pharmacology indicated that amongst several endogenous biogenic amines, including dopamine, melatonin, epinephrine, norepinephrine or histamine, only 5-HT (serotonin) was capable of completely displacing [¹²⁵I]-LSD binding, exhibiting a Kᵢ of 150 nM (Figure 2 and Table 1). The Hill coefficient for the 5-HT competition curve was not significantly different from unity and the affinity of 5-HT was not influenced by addition of the guanine nucleotide analogue GppNHp.

The binding of 5-HT to St-B17 was also investigated directly using [³H]-5-HT as the radioligand. Saturation analysis of [³H]-5-HT binding revealed the presence of a single class of high affinity, saturable binding sites in transfected, but not untransfected, Cos-7 cells. Analysis of the [³H]-5-HT saturation binding data revealed a K_{D} of 37 (±5.0, n=3) nM and Bₘₐₓ values of 1-3 pMoles/mg protein. The binding of [³H]-5-HT was similarly not affected by the addition of guanine nucleotides. These initial binding data would thus suggest than St-B17 encodes a 5-HT receptor subtype.

Further characterization of the St-B17 pharmacology involved the utilization of a variety of drugs which are known to exhibit specificity for various serotonergic receptor subtypes and other binding sites. The average Kᵢ values for compounds competing with better than 10 µM affinity are shown in Table 1 with representative competition curves for [¹²⁵I]-LSD binding shown in Figure 2.

Examination of the rank order of potency for a variety of serotonergic agents reveals that the pharmacology of clone St-B17 does not correspond to any previously described serotonin receptor subtype. A number of drugs selective for 5-HT₃ and 5-HT₄ receptors (i.e., MDL 72222, ICS 205,930, or DAU 6285) exhibit virtually no affinity for St-B17, while agents selective for other 5-HT receptor subtypes such as 8-OH-DPAT (5-HT_{1A}), CGS 12066B (5-HT_{1B}), mesulergine (5-HT_{1C}), or ketanserin (5-HT₂) bind with relatively low affinity. Ergot alkaloids, especially ergoline derivatives (i.e., LSD, lisuride, or pergolide), display relatively high affinity for St-B17 as does the non-selective serotonergic antagonist methiothepin.

Interestingly, the atypical and typical anti-psychotics clozapine and loxapine, respectively, also exhibited high affinity for St-B17, as did several tricyclic anti-depressant drugs (i.e., amoxipine, clomiprimine, and amitriptyline) which all had Kᵢ values under 100 nM. In general, the drugs which exhibited the greatest affinity for St-B17 (i.e., Kᵢ<100 nM) were tricyclic, ergoline or tryptamine derivatives.

Competition for [³H]-5-HT binding by a number of drugs revealed, with a few exceptions, the same rank order of potency as for inhibition of [¹²⁵I]-LSD binding (Table 1). However, for some drugs the Kᵢ values determined by competition with [³H]-5-HT were up to 5-fold lower than those determined by competition with [¹²⁵I]-LSD, with the exception of clozapine, metergoline and amitriptyline, which exhibited somewhat greater potency in competition with [¹²⁵I]-LSD.

Although the pharmacological profile of St-B17 did not correspond to previously defined 5-HT receptor subtypes, it did resemble the profile described by Conner and Mansour for 5-HT stimulation of adenylyl cyclase activity in the NCB-20 neuroblastoma cell line (Mol. Pharmacol. (1990) **37**, 742-751). In these cells 5-HT activated adenylyl cyclase with an EC₅₀ of 300 nM and a variety of tryptamine derivatives exhibit EC₅₀ values similar to their Kᵢ values for inhibition of [¹²⁵I]-LSD binding to St-B17. Similarly, antagonism of 5-HT-stimulated adenylyl cyclase activity in NCB-20 cells by a variety of compounds, including methiothepin, mianserin, amitriptyline, and cyproheptadine, also exhibited apparent Kᵢ values and a rank order of potency similar to those observed for inhibition of [¹²⁵I]-LSD binding to St-B17.

It is contemplated that other means of labeling LSD or the other tested drugs would also provide an assay for the inhibition of compounds such as that discussed above. LSD could be labeled either radioactively or colormetrically and still provide the desired assay.

The distribution of mRNA encoding St-B17 was evaluated by Northern blot analysis of poly (A+) RNA prepared from a variety of rat brain regions as well as other peripheral tissues. The results from these experiments are shown below in Example 7.

### Example 7: Distribution of mRNA Encoding St-B17

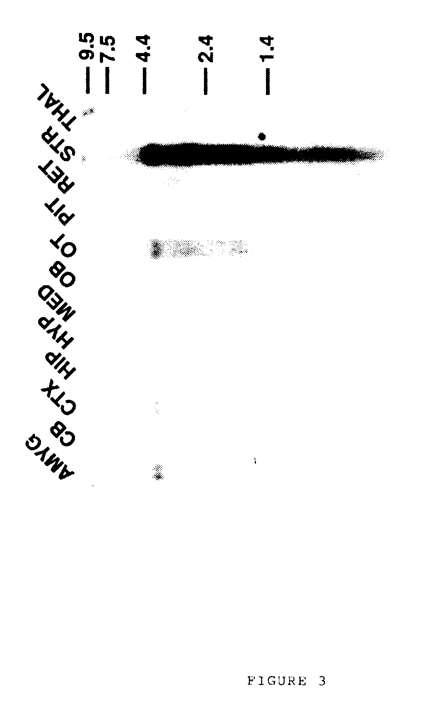

Figure 3 shows a Northern blot analysis of St-B17 RNA transcripts in rat brain. Each lane contained 3 µg of poly (A+) RNA. Lanes: AMYG, amygdala; CB, cerebellum; CTX, cerebral cortex; HIP, hippocampus; HYP, hypothalamus; MED, medulla; OB, olfactory bulb; OT, olfactory tubercle; PIT, pituitary; RET, retina; STR, striatum; THAL, thalamus. Locations of RNA size (kb) markers are indicated.

To prepare the Northern blot, poly (A+) RNA was prepared from rat brain regions using the FASTTRACK© mRNA isolation system (INVITROGEN). 3 µg of poly (A+) RNA was denatured and subjected to electrophoresis on a 1% agarose gel containing 0.66 M formaldehyde. RNA was transferred to a nylon membrane (Genescreen Plus, Dupont) by capillary transfer and immobilized by UV crosslinking. Northern blots were probed with the entire 2.8 kb cDNA insert of clone St-B17 [³²P]-labeled by the random primer method. Northern blots were hybridized with 3 x 10⁶ dpm/ml of probe in 1 M NaCl, 1% SDS, 10% dextran sulfate at 60°C for 18 hr. Blots were washed in 2X SSC/1% SDS at room temperature, followed by 0.2X SSC/1% SDS at 65°C, and were exposed with an intensifying screen for 15 days at -70°C.

A single transcript of 4.2 kb was observed in various brain regions with highest expression appearing to occur in the corpus striatum (Figure 3). St-B17 mRNA was also observed in the amygdala, cerebral cortex and olfactory tubercle whereas it was absent, or present in undetectable levels, in the cerebellum, hippocampus, hypothalamus, medulla, olfactory bulb, pituitary, retina and thalamus. Similarly, no transcript was observed, even after prolonged exposures, in Northern blots of mRNA from rat heart, lung, kidney, spleen, pancreas, smooth muscle, skeletal muscle, stomach, ovary, prostrate, or testes (data not shown).

This result indicates that the St-B17 receptor is produced in much higher levels in the corpus striatum than in other regions of the brain. In addition, the receptor is undetectable in non-brain tissues.

The localization of St-B17 mRNA to limbic and cortical regions, and the relatively potent interaction of several therapeutically important drugs, including the atypical anti-psychotic clozapine, the typical anti-psychotic loxapine, and the tricyclic anti-depressants amoxipine, clomiprimine, amitriptyline, and nortriptyline with St-B17 suggests that this receptor may play an important though hitherto unappreciated role in several neuropsychiatric disorders which involve serotonergic systems.

In addition to the mRNA distribution of St-B17, we were also interested in the intracellular cascade of events following agonist binding to the receptor. One possible mechanism of receptor signaling involved adenylate cyclase activity. To investigate this possibility, the following experiment was performed.

### Example 8: Analysis of Receptor Mediated cAMP Activity

Human Embryonic Kidney cells (HEK 293 available from the ATCC) were stably co-transfected in 150mm dishes by the CaPO₄ technique with 30 µg of pSRα-B17 and 3µg of pMam-neo (INVITROGEN). These HEK 293 cells were then subjected to selection with 600 µg/ml genetecin (G418) (GIBCO). The resistant colonies were collected and screened for receptor expression by [¹²⁵I]-LSD binding as described in Example 6. One clone was isolated which expressed approximately 800 fMoles/mg St-B17 receptor protein and was subsequently used for assessment of cAMP accumulation.

Intracellular cAMP levels were determined after a 5 minute incubation of intact cells with various test compounds at 37°C in the presence of the cAMP phosphodiesterase inhibitor RO-20-1724, using a modification of a previously described cAMP assay (Monsma, F.J., Jr., Barton, A.C., and Sibley, D.R. (1990) J. Neurochemistry **54**, 1200-1207) wherein the adrenal cAMP binding protein was replaced with 0.8mg/ml protein kinase A in water.

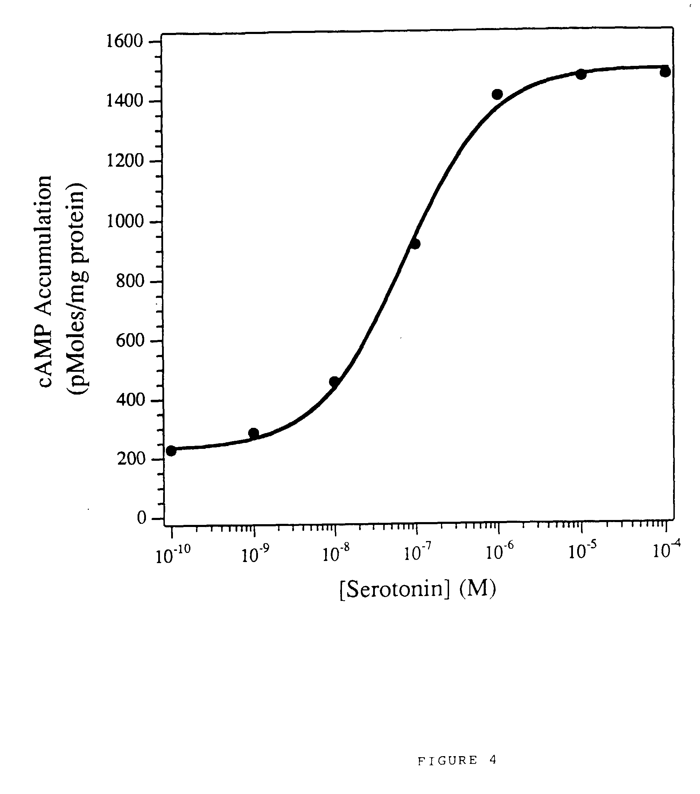

As shown in Figure 4, serotonin causes a potent dose-dependent increase in cAMP levels in transfected HEK-293 cells with an average 50% effective concentration (EC₅₀) of 145 (±40, n=3) nM, whereas there was no detectable response in untransfected cells (data not shown) . This suggests that the St-B17 receptor mediates some part of its cell signaling through a cAMP-related pathway.

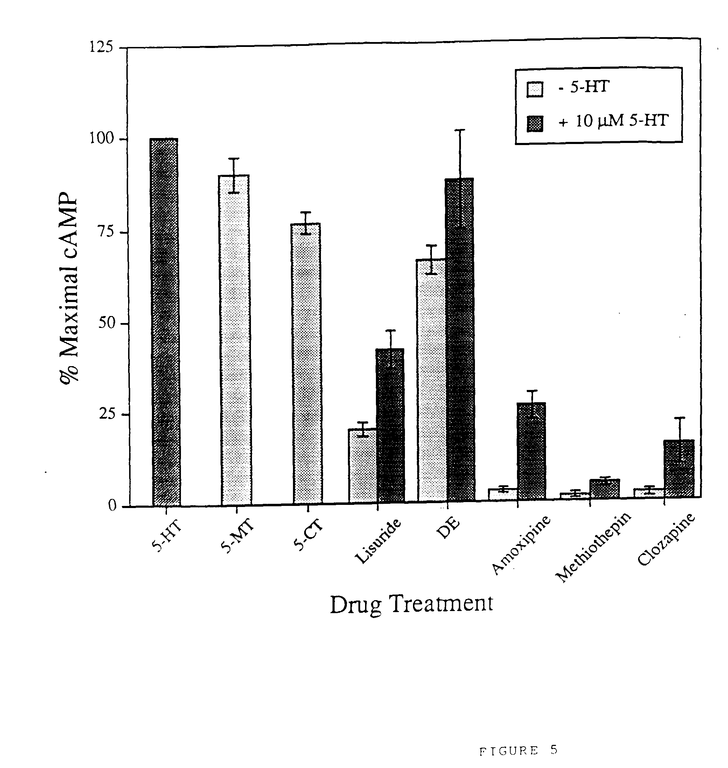

A pharmacological analysis of the cAMP response (Figure 5) indicates that the serotonergic agonists 5-MT and 5-CT are also able to elicit an increase in cAMP levels. The ergot alkaloids lisuride and dihydroergotamine also stimulate cAMP accumulation, although these drugs appear to function as partial agonists to the St-B17 receptor. Amoxipine, methiothepin and clozapine (Figure 5) all appear to act as antagonists of this receptor as they had no significant effect on cAMP levels on their own, but were able to substantially inhibit the response elicited by 5-HT. These data suggest that the St-B17 receptor is functionally linked to the adenylyl cyclase signal transduction system.

The measurement of cAMP levels in a cell transfected with St-B17 can thereby provide an assay for detecting potential agonists and antagonists of serotonin receptors. Using the above method, we can analyze the accumulated level of cAMP in the cell after treatment with a drug to be tested. A lowering of the cAMP level in the presence of 5-HT indicates a drug which has inhibitory affects on 5-HT receptor binding.

We were also interested in isolating the human variation of the present invention. Our method of isolating the human receptor is discussed below.

### Example 9: Isolation of the Human St-B17 Receptor

Two different restriction fragments derived from the rat St-B17 gene were used as probes to retrieve the human homolog. A 1192 base-pair Xma I-BstXI fragment and a 655 base-pair Bam HI-EagI fragment of rat St-B17 were labeled with [α³²P]-dCTP using the well known nick-translation method. These labeled fragments were then used to screen a commercially available human genomic library (Stratagene #946205).

The library was plated onto host cells C600 and PLK17 respectively, and duplicate nylon (Nytran, Schleicher and Schuell) lifts made. Each of the labeled fragments was hybridized (6 X SSC at 55°C, pH7) and washed (2 X SSC, 58°C) at moderate stringency. Positive isolates were plaque purified through a second round of hybridization.

An insert from each positive plaque isolate was excised by EcoRI digestion, and then subcloned into a corresponding site in the phagemid vector pBluescript SK(-) (Stratagene). These inserts were then digested with several restriction enzymes, and run on a Southern Blot. Fragments hybridizing to labeled St-B17 rat sequences were identified by Southern hybridization. Hybridizing fragments were then subcloned into pBluescript SK(-) as discussed above. The nucleic acid sequence of these inserts was then determined by dideoxynucleotide termination using Sequenase (United States Biochemicals) in conjunction with standard subcloning techniques. The nucleotide sequence of the human St-B17 gene is SEQUENCE ID NO:12.

It should be understood that all of the previous experiments relating to identification of binding ligands to the rat receptor can be performed in a similar fashion with the human gene. For instance, the human gene can be cloned into an expression plasmid and placed in COS-7 cells by methods similar to those discussed above, and used to assay binding of competitors such as those revealed in Table 1. This would provide a method of assaying inhibitors of serotonin binding to the St-B17 receptor.

Specifically, Example 8 can be repeated with the human receptor to reveal if a particular compound was mimicking or inhibiting the binding of serotonin by measurement of the relative level of cAMP before and after treatment with the compound. The methods of this example can also be repeated with cDNA of other mammals or other vertebrates to obtain the corresponding St-B17 receptor from these organisms.

Other experiments are also contemplated relating to discovering which cells in vivo express the receptor gene on their surface. To allow us the ability to study the expression of the receptor on cell surfaces throughout the body, we needed to produce antibodies against the receptor protein. A method for producing antibodies is discussed below in Example 10.

### Example 10: Production of Antibodies Against St-B17

COS-7 cells expressing the St-B17 receptor protein derived in Examples 11 and 12 are lysed with NP40, and the isolated membranes are injected into rabbits. The lysed membranes are isolated in a non-ionic detergent so as not to affect the membrane bound receptors. Freunds adjuvant is used in the injection to help stimulate an antigenic response by the rabbits. After two booster shots of the lysed membranes, the rabbits are bled and the sera isolated by centrifugation.

The antibodies in the crude rabbit sera extract are ¹²⁵I labeled by well known methods, and tested for activity against the transfected COS-7 cells. A western blot having one lane containing proteins from transfected cell lysates, and a second lane having untransfected lysates (control) is run. A strong band indicating antibody binding at 46.8kd in the transfected cell lane, that is not apparent in the untransfected lane shows that polyclonal antibodies against the St-B17 receptor protein have been properly isolated.

Monoclonal antibodies can be made by well known methods in addition to the polyclonal antibodies discussed above. One method of producing monoclonal antibodies is discussed below in Example 11.

### Example 11: Production of Monoclonal Antibodies Against St-B17

The St-B17 transfected COS-7 cells produced in the previous examples are lysed with NP-40 and the cell membranes are pelleted by centrifugation. The isolated membranes, having bound St-B17 receptor proteins are injected in Freunds adjuvant into mice. After being injected 9 times over a three week period, the mice spleens are removed and resuspended in PBS.

The suspended spleen cells are mixed (approximately 4:1) with SP 2/0 Myeloma cells. Polyethylene glycol is added to fuse the myeloma cells to the spleen cells, and the fused cells are selected in HAT media. The fused cells are aliquoted so that only one cell is grown in each well of a 96 well microtiter plate. Each cell is grown and the media removed and secreted proteins are ¹²⁵I labeled. The labeled media from each well is used to probe a Western blot of transfected and untransfected COS-7 cells (see Example 12). The desired fusion cell will produce a monoclonal antibody that strongly binds a 46.8kD band in the transfected COS-7 cell lane on the Western blot, but doesn't bind to any other protein in that lane, or the control lane.

This method provides a way of detecting expression of the St-B17 serotonin receptor protein. Another method of detecting expression of St-B17 is by in situ hybridization as discussed below in Example 12.

### Example 12: In Situ Hybridization of St-B17

In situ hybridization allows the identification of mRNA within intact tissues, such as the rat brain. In this method, oligonucleotides corresponding to unique portions of the St-B17 gene (SEQ ID NO: 7) are used to detect specific mRNA species in the brain.

An anesthetized rat is transcardially perfused with cold PBS (5-20 minutes), followed by perfusion with a 4% formaldehyde solution. The brain is removed, frozen in liquid nitrogen, and cut into 5 µm to 30 µm sections. The sections are placed on slides and incubated in proteinase K for approximately 15 minutes. The slides are then rinsed in DEP, water, and ethanol, and placed in a prehybridization buffer.

A radioactive probe corresponding to primer Pl is made by nick translation and incubated with the sectioned brain tissue. After incubation and air drying, the labeled areas are visualized by autoradiography. Dark spots on the tissue sample indicate hybridization of the probe with the brain mRNA thereby demonstrating expression of the St-B17 receptor.

All of the above cited references are herein incorporated by reference. The present invention should not be limited by these examples but only by the following claims.

## Claims

1. A nucleotide sequence encoding a mammalian serotonin receptor protein St-B17, said protein exhibiting high affinity binding for clozapine, loxapine and amoxipine and exhibiting high expression levels in limbic and cortical regions of the brain, said nucleotide sequence being selected from
(a) a nucleotide sequence comprising SEQ ID NO. 7;
(b) a nucleotide sequence comprising SEQ ID NO. 12;
(c) a nucleotide sequence hybridizing under moderate stringency conditions at 6XSSC and 55°C, pH7, to a 1192 bp XmaI-BstXI or a 655 bp BamHI-EagI fragment from SEQ ID NO:7; or
(d) a nucleotide sequence encoding a protein having the amino acid sequence shown by SEQ ID NO. 8 or SEQ ID NO. 13.

2. The nucleotide sequence according to claim 1, wherein said protein is human ST-B17.

3. A nucleotide sequence having at least 30 contiguous nucleotides of the nucleotide sequence according to claim 1(a) or (b).

4. A recombinant construct comprising the nucleotide sequence according to claim 1 or 2, operably linked to a heterologous promoter.

5. The recombinant construct according to claim 4, which is an expression vector.

6. The recombinant construct according to claim 5, which is a eukaryotic expression vector.

7. The recombinant construct according to claim 6, wherein the eukaryotic expression vector is pSRα-B17, which is configured to express a fusion gene having a functional 5' leader and initiation codon, and a correctly spliced intron/exon junction.

8. A mammalian cell line comprising the nucleotide sequence of Claim 1 or 2 or the vector of any one of Claims 5 to 7, said mammalian cell line expressing St-B17 serotonin receptor.

9. The cell line of Claim 8, wherein said cells are derived from a human.

10. The cell line of Claim 8, wherein said cells are HEK 293.

11. A protein encoded by the nucleotide sequence of Claim 1 or 2, the recombinant construct of any one of Claims 4 to 7 or produced by the cell line of any one of claims 8 to 10.

12. An antibody specifically directed against the protein of Claim 11.

13. The antibody of Claim 12 which is a monoclonal antibody.

14. The antibody of Claim 12 which is a polyclonal antibody.

15. A method of screening a drug candidate for central nervous system activity, comprising the steps of contacting said drug candidite with the protein of Claim 11, and measuring binding of said drug candidate by said protein.

16. A method for screening a drug candidate for central nervous system activity, comprising the steps of
contacting said protein of Claim 11 with a first molecule known to be bound by said protein to form a first complex of said protein and said first molecule;
contacting said first complex with said drug candidate; and
measuring whether said drug candidate displaces said first molecule from said first complex.

17. The method of Claim 16, wherein said measuring step comprises measuring the formation of a second complex of said protein and said drug candidate.

18. The method of Claim 16, wherein said measuring step comprises measuring the amount of said first molecule that is not bound to said protein.

19. A method of screening a ligand for binding to the St-B17 serotonin receptor protein of Claim 11, said method comprising the steps of
transfecting a cell line with a gene encoding the St-B17 serotonin receptor protein in the expression vector of any one of Claims 5 to 7;
culturing said cell line to express said gene in media containing a ligand of said receptor; and
measuring the binding of said ligand to said receptor produced by said cell line.

20. The method of Claim 19, wherein said cell line is from a mammal, preferably HEK 293.

21. The method of Claim 19 or 20, wherein said ligand is labeled.

22. The method of Claim 21, wherein said label is radioactive.

23. The method of Claim 21, wherein said label is colorimetric.

24. A method of determining the ability of a drug to inhibit ligand binding to the St-B17 serotonin receptor protein of Claim 11, the method comprising
transfecting a cell line with a DNA sequence encoding St-B17 in the expresion vector of any one of Claims 5 to 7;
culturing said cell line to express the St-B17 receptor in media containing a ligand of said receptor;
determining the level of binding of said ligand to said receptor;
culturing the same cell line to express said receptor in the presence of both said ligand and said drug; and
determining the level of binding of said ligand to said expressed receptor, wherein a lower level of binding in the presence of said drug indicates that said compound is an inhibitor of ligand binding.

25. The method of claim 24, wherein said cell line is mammalian, preferably HEK 293.

26. The method of Claim 24 or 25, wherein said ligand is labeled.

27. The method of Claim 24 or 25, wherein said drug is labeled.

28. The method of any of Claim 24 to 27, wherein said ligand is serotonin.

29. The method of any one of claims 24 to 28, wherein said expression vector is configured to express a fusion gene having a functional 5' leader and initiation codon, and a correctly spliced intron/exon junction.

30. A method for determining the ability of a drug to inhibit ligand binding to the St-B17 serotonin receptor of Claim, 11, the method comprising:
transfecting a cell line with a gene encoding the St-B17 receptor protein in an expression vector;
culturing said cell line to express said receptor;
determining the level of binding of said ligand to said receptor;
culturing the same cell line to express said receptor in the presence of both said ligand and the drug being tested for inhibition; and
determining the level of cAMP accumulation in said cell, wherein a lower level of cAMP accumulation in said cell in the presence of said drug indicates inhibition of ligand binding.

31. A method of determining the ability of a drug to inhibit ligand binding to the St-B17 serotonin receptor protein of Claim 11, the method comprising
transfecting a cell line with a DNA sequence encoding St-B17 in an expression vector;
culturing said cell line to express the St-B17 receptor in media containing a ligand of said receptor;
isolating the membranes from said cell line, wherein the membranes contain the expressed St-B17 receptor;
determining the level of binding of said ligand to said receptor on said membranes;
incubating said membranes in the presence of both said ligand and said drug; and
determining the level of binding of said ligand to said membranes, wherein a lower level of binding in the presence of said drug indicates that said compound is an inhibitor of ligand binding.

32. A. method for detecting expression of a polynucleotide encoding the St-B17 serotonin receptor of Claim 11 in a tissue sample, comprising the steps of
removing said tissue sample from a mammal;
contacting the mRNA in the cytoplasm of said tissue sample with a labeled polynucleotide probe having homology to said polynucleotide encoding said St-B17 serotonin receptor; and
detecting the binding of said probe with said tissue, wherein positive binding indicates expression of the St-B17 serotonin receptor protein.

## Patentansprüche

1. Nucleotidsequenz, die ein Säuger-Serotoninrezeptor-Protein St-B17 codiert, wobei das Protein eine hohe Bindungsaffinität für Clozapin, Loxapin und Amoxipin und eine hohe Expressionsrate in Limbus und Rindenregion des Gehirns aufweist, wobei die Nucleotidsequenz ausgewählt ist aus
(a) einer Nucleotidsequenz umfassend SEQ ID No. 7;
(b) einer Nucleotidsequenz umfassend SEQ ID No. 12;
(c) einer Nucleotidsequenz, die unter moderaten stringenten Bedingungen bei 6xSSC und 55°C, pH 7, an ein 1192 bp-XmaI-BstXI- oder einem 655 bp-BamHI-EagI-Fragment von SEQ ID No.7 hybridisiert; oder
(d) einer Nucleotidsequenz, die ein Protein codiert, das die in SEQ ID No. 8 oder SEQ ID No. 13 abgebildete Aminosäuresequenz hat.

2. Nucleotidsequenz nach Anspruch 1, wobei das Protein menschliches ST-B17 ist.

3. Nucleotidsequenz, die wenigstens 30 benachbarte Nucleotide der Nucleotidsequenz nach Anspruch 1 (a) oder (b) hat.

4. Rekombinantes Konstrukt, umfassend die Nucleotidsequenz nach Anspruch 1 oder 2 funktionell verknüpft mit einem heterologen Promotor.

5. Rekombinantes Konstrukt nach Anspruch 4, das ein Expressionsvektor ist.

6. Rekombinantes Konstrukt nach Anspruch 5, das ein eukaryotischer Expressionsvektor ist.

7. Rekombinantes Konstrukt nach Anspruch 6, wobei der eukaryotische Expressionsvektor pSRα-B17 ist, der so konfiguriert ist, daß er ein Fusionsgen exprimiert, das einen funktionellen 5'-Leader und ein Initiationscodon sowie eine korrekt gespleißte Intron/Exon-Verknüpfung hat.

8. Säugerzellinie, umfassend die Nucleotidsequenz nach Anspruch 1 oder 2 oder den Vektor nach einem der Ansprüche 5 bis 7, wobei die Säugerzellinie den St-B17-Serotoninrezeptor exprimiert.

9. Zellinie nach Anspruch 8, wobei die Zellen vom Menschen abstammen.

10. Zellinie nach Anspruch 8, wobei die Zellen HEK 293 sind.

11. Protein, codiert durch eine Nucleotidsequenz nach Anspruch 1 oder 2, das rekombinante Konstrukt nach einem der Ansprüche 4 bis 7 oder hergestellt durch die Zellinie nach einem der Ansprüche 8 bis 10.

12. Antikörper, der spezifisch gegen das Protein nach Anspruch 11 gerichtet ist.

13. Antikörper nach Anspruch 12, der ein monoclonaler Antikörper ist.

14. Antikörper nach Anspruch 12, der ein polyclonaler Antikörper ist.

15. Verfahren zur Durchmusterung eines Arzneistoffkandidaten auf Aktivität im zentralen Nervensystem, umfassend die Schritte des Inkontaktbringens des Arzneistoffkandidaten mit dem Protein nach Anspruch 11 und Messung der Bindung des Arzneistoffkandidatens durch das Protein.

16. Verfahren zur Durchmusterung eines Arzneistoffkandidatens auf Aktivität im zentralen Nervensystem, umfassend die Schritte:
Inkontaktbringen des Proteins nach Anspruch 11 mit einem ersten Molekül, von dem bekannt ist, daß es durch das Protein gebunden wird, um einen ersten Komplex aus dem Protein und dem ersten Molekül zu bilden,
Inkontaktbringen des ersten Komplex mit dem Arzneistoffkandidaten; und
Messung, ob der Arzneistoffkandidat das erste Molekül aus dem ersten Komplex verdrängt.

17. Verfahren nach Anspruch 16, wobei der Meßschritt die Messung der Bildung eines zweiten Komplexes des Proteins und des Arzneistoffkandidaten umfaßt.

18. Verfahren nach Anspruch 16, wobei der Meßschritt die Messung der Menge des ersten Moleküls umfaßt, das nicht an das Protein gebunden ist.

19. Verfahren zur Durchmusterung eines Liganden auf die Bindung des St-B17-Serotoninrezeptorproteins nach Anspruch 11, wobei das Verfahren die Schritte umfaßt:
Transfektion einer Zellinie mit einem Gen, das das St-B17-Serotoninrezeptorprotein in dem Expressionsvektor nach einem der Ansprüche 5 bis 7 codiert;
Züchten der Zellinie, um das Gen in einem Medium zu exprimieren, das einen Liganden des Rezeptors enthält; und
Messung der Bindung des Liganden an den durch die Zellinie produzierten Rezeptor.

20. Verfahren nach Anspruch 19, wobei die Zellinie von einem Säuger stammt, vorzugsweise HEK 293.

21. Verfahren nach Anspruch 19 oder 20, wobei der Ligand markiert ist.

22. Verfahren nach Anspruch 21, wobei der Marker radioaktiv ist.

23. Verfahren nach Anspruch 21, wobei der Marker kolorimetrisch ist.

24. Verfahren zur Bestimmung der Eigenschaft eines Arzneistoffes, die Ligandenbindung an das St-B17-Serotonin-Rezeptorprotein nach Anspruch 11 zu hemmen, umfassend
Transfektion einer Zellinie mit einer DNA-Sequenz, die St-B17 in dem Expressionsvektor nach einem der Ansprüche 5 bis 7 codiert;
Züchten der Zellinie, um den St-B17-Rezeptor in einem Medium zu exprimieren, das einen Liganden des Rezeptors enthält;
Bestimmung des Bindungsgrads des Liganden an den Rezeptor;
Züchten der gleichen Zellinie zur Expression des Rezeptors in der Gegenwart sowohl des Liganden als auch des Arzneistoffes; und
Bestimmung des Bindungsgrads des Liganden an den exprimierten Rezeptor, wobei ein geringerer Bindungsgrad in Gegenwart des Arzneistoffes anzeigt, daß die Verbindung ein Inhibitor der Ligandenbindung ist.

25. Verfahren nach Anspruch 24, wobei die Zellinie von einem Säuger stammt, vorzugsweise HEK 293.

26. Verfahren nach Anspruch 24 oder 25, wobei der Ligand markiert ist.

27. Verfahren nach Anspruch 24 oder 25, wobei der Arzneistoff markiert ist.

28. Verfahren nach einem der Ansprüche 24 bis 27, wobei der Ligand Serotonin ist.

29. Verfahren nach einem der Ansprüche 24 bis 28, wobei der Expressionsvektor so konfiguriert ist, daß er ein Fusionsgen exprimiert, das einen funktionellen 5'-Leader hat und ein Initiationscodon sowie eine korrekt gespleißte Intron/Exon-Verknüpfung hat.

30. Verfahren zur Bestimmung der Eigenschaft eines Arzneistoffes, die Ligandenbindung an den St-B17-Serotoninrezeptor nach Anspruch 11 zu hemmen, umfassend:
Transfektion einer Zellinie mit einem Gen, das das St-B17-Rezeptorprotein in einem Expressionsvektor codiert;
Züchten der Zellinie, um den Rezeptor zu exprimieren;
Bestimmung des Bindungsgrads des Liganden an den Rezeptor;
Züchten der gleichen Zellinie zur Expression des Rezeptors in der Gegenwart sowohl des Liganden als auch des Arzneistoffes, der für die Hemmung getestet wird, und
Bestimmung der Höhe des cAMP-Akkumulation in der Zelle, wobei ein geringerer Grad an cAMP-Akkumulation in der Zelle in der Gegenwart des Arzneistoffes die Hemmung der Ligandenbindung anzeigt.

31. Verfahren zur Bestimmung der Eigenschaft eines Arzneistoffes, die Ligandenbindung an das St-817-Serotonin-Rezeptorprotein nach Anspruch 11 zu hemmen, umfassend: Transfektion einer Zellinie mit einer DNA-Sequenz, die St-B17 in einem Expressionsvektor codiert;
Züchten der Zellinie zur Expression des St-B17-Rezeptors in einem Medium, das einen Liganden des Rezeptors enthält,
Isolieren der Membranen von der Zellinie, wobei die Membranen den exprimierten St-B17-Rezeptor enthalten,
Bestimmung des Bindungsgrads des Liganden an den Rezeptor auf den Membranen;
Inkubation der Membranen in der Gegenwart sowohl des Liganden als auch des Arzneistoffes; und
Bestimmung des Bindungsgrads des Liganden an die Membranen, wobei ein geringerer Bindungsgrad in der Gegenwart des Arzneistoffes anzeigt, daß die Verbindung ein Inhibitor der Ligandenbindung ist.

32. Verfahren zum Nachweis der Expression eines Polynucleotids, das den St-B17-Serotoninrezeptor nach Anspruch 11 codiert in einer Gewebeprobe, umfassend die Schritte
Entnahme einer Gewebeprobe von einem Säuger;
Inkontaktbringen der mRNA im Cytoplasma der Gewebeprobe mit einer markierten Polynucleotidsonde, die Homologie zu dem Polynucleotid besitzt, das den St-B17-Serotonin-Rezeptor codiert; und
Nachweis der Bindung der Sonde mit dem Gewebe, wobei eine positive Bindung die Expression des St-B17-Serotonin-Rezeptorproteins anzeigt.

## Revendications

1. Séquence nucléotidique codant une protéine récepteur de sérotonine de mammifère St-B17, ladite protéine présentant une liaison à haute affinité pour la clozapine, la loxapine et l'amoxipine et présentant de hauts niveaux d'expression dans les régions limbique et corticale du cerveau, ladite séquence nucléotidique étant choisie parmi
(a) une séquence nucléotidique comprenant SEQ ID NO. 7;
(b) une séquence nucléotidique comprenant SEQ ID NO. 12;
(c) une séquence nucléotidique s'hybridant dans des conditions de stringence modérée à 6XSSC et 55°C, pH 7, avec un fragment XmaI-BstXI de 1192 pb ou un fragment BamHI-EagI de 655 pb de SEQ ID NO : 7 ; ou
(d) une séquence nucléotidique codant une protéine ayant la séquence d'acides aminés indiquée par SEQ ID NO. 8 ou SEQ ID NO. 13.

2. Séquence nucléotidique selon la revendication 1, dans laquelle ladite protéine est St-B17 humaine.

3. Séquence nucléotidique ayant au moins 30 nucléotides contigus de la séquence nucléotidique selon la revendication 1(a) ou (b).

4. Construction recombinée comprenant la séquence nucléotidique selon la revendication 1 ou 2, liée de manière active à un promoteur hétérologue.

5. Construction recombinée selon la revendication 4 qui est un vecteur d'expression.

6. Construction recombinée selon la revendication 5 qui est un vecteur d'expression eucaryote.

7. Construction recombinée selon la revendication 6, dans laquelle le vecteur d'expression eucaryote est pSRα-B17 qui est configuré pour exprimer un gène de fusion ayant un leader en 5' et un codon d'initiation fonctionnels, et une jonction intron/exon épissée correctement.

8. Lignée cellulaire de mammifère comprenant la séquence nucléotidique selon la revendication 1 ou 2 ou le vecteur selon l'une quelconque des revendications 5 à 7, ladite lignée cellulaire de mammifère exprimant le récepteur de sérotonine St-B17.

9. Lignée cellulaire selon la revendication 8, dans laquelle lesdites cellules sont issues d'un humain.

10. Lignée cellulaire selon la revendication 8, dans laquelle lesdites cellules sont HEK 293.

11. Protéine codée par la séquence nucléotidique selon la revendication 1 ou 2, la construction recombinée selon l'une quelconque des revendications 4 à 7 ou produite par la lignée cellulaire selon l'une quelconque des revendications 8 à 10.

12. Anticorps dirigé spécifiquement contre la protéine selon la revendication 11.

13. Anticorps selon la revendication 12 qui est un anticorps monoclonal.

14. Anticorps selon la revendication 12 qui est un anticorps polyclonal.

15. Procédé de sélection d'un candidat médicament concernant une activité sur le système nerveux central, comprenant les étapes de mise en contact dudit candidat médicament avec la protéine selon la revendication 11 et de mesure de la liaison dudit candidat médicament par ladite protéine.

16. Procédé pour sélectionner un candidat médicament concernant une activité sur le système nerveux central comprenant les étapes consistant à
mettre en contact ladite protéine selon la revendication 11 avec une première molécule dont on sait qu'elle est liée par ladite protéine pour former un premier complexe de ladite protéine et de ladite première molécule ;
mettre en contact ledit premier complexe avec ledit candidat médicament ; et
mesurer si ledit candidat médicament déplace ladite première molécule dudit premier complexe.

17. Procédé selon la revendication 16, dans lequel ladite étape de mesure comprend la mesure de la formation d'un second complexe de ladite protéine et dudit candidat médicament.

18. Procédé selon la revendication 16, dans lequel ladite étape de mesure comprend la mesure de la quantité de ladite première molécule qui n'est pas liée à ladite protéine.

19. Procédé de sélection d'un ligand concernant la liaison à la protéine récepteur de sérotonine St-B17 selon la revendication 11, ledit procédé comprenant les étapes de
transfection d'une lignée cellulaire avec un gène codant la protéine récepteur de sérotonine St-B17 dans le vecteur d'expression selon l'une quelconque des revendications 5 à 7;
culture de ladite lignée cellulaire pour exprimer ledit gène dans un milieu contenant un ligand dudit récepteur; et
mesure de la liaison dudit ligand audit récepteur produit par ladite lignée cellulaire.

20. Procédé selon la revendication 19, dans lequel ladite lignée cellulaire est issue d'un mammifère, et est de préférence HEK 293.

21. Procédé selon la revendication 19 ou 20, dans lequel ledit ligand est marqué.

22. Procédé selon la revendication 21, dans lequel ledit marqueur est radioactif.

23. Procédé selon la revendication 21, dans lequel ledit marqueur est colorimétrique.

24. Procédé de détermination de l'aptitude d'un médicament à inhiber la liaison d'un ligand à la protéine récepteur de sérotonine St-B17 selon la revendication 11, le procédé comprenant
la transfection d'une lignée cellulaire avec une séquence d'ADN codant St-B17 dans le vecteur d'expression selon l'une quelconque des revendications 5 à 7 ;
la culture de ladite lignée cellulaire pour exprimer le récepteur St-B17 dans un milieu contenant un ligand dudit récepteur ;
la détermination du niveau de liaison dudit ligand audit récepteur ;
la culture de la même lignée cellulaire pour exprimer ledit récepteur en présence dudit ligand et dudit médicament ; et
la détermination du niveau de liaison dudit ligand audit récepteur exprimé, un niveau de liaison plus bas en présence dudit médicament indiquant que ledit composé est un inhibiteur de la liaison du ligand.

25. Procédé selon la revendication 24, dans lequel ladite lignée cellulaire est de mammifère, et est de préférence HEK 293.

26. Procédé selon la revendication 24 ou 25, dans lequel ledit ligand est marqué.

27. Procédé selon la revendication 24 ou 25, dans lequel ledit médicament est marqué.

28. Procédé selon l'une quelconque des revendications 24 à 27, dans lequel ledit ligand est la sérotonine.

29. Procédé selon l'une quelconque des revendications 24 à 28, dans lequel ledit vecteur d'expression est configuré pour exprimer un gène de fusion ayant un leader en 5' et un codon d'initiation fonctionnels, et une jonction intron/ exon épissée correctement.

30. Procédé de détermination de l'aptitude d'un médicament à inhiber la liaison d'un ligand au récepteur de sérotonine St-B17 selon la revendication 11, le procédé comprenant :
la transfection d'une lignée cellulaire avec un gène codant la protéine récepteur St-B 17 dans un vecteur d'expression ;
la culture de ladite lignée cellulaire pour exprimer ledit récepteur ;
la détermination du niveau de liaison dudit ligand audit récepteur ;
la culture de la même lignée cellulaire pour exprimer ledit récepteur en présence dudit ligand et du médicament testé pour l'inhibition ; et
la détermination du niveau d'accumulation d'AMPc dans ladite cellule, un niveau d'accumulation d'AMPc plus bas dans ladite cellule en présence dudit médicament indiquant une inhibition de la liaison du ligand.

31. Procédé de détermination de l'aptitude d'un médicament à inhiber la liaison d'un ligand à la protéine récepteur de sérotonine St-B17 selon la revendication 11, le procédé comprenant
la transfection d'une lignée cellulaire avec une séquence d'ADN codant St-B 17 dans un vecteur d'expression ;
la culture de ladite lignée cellulaire pour exprimer le récepteur St-B17 dans un milieu contenant un ligand dudit récepteur ;
l'isolement des membranes à partir de ladite lignée cellulaire, les membranes contenant le récepteur St-B 17 exprimé ;
la détermination du niveau de liaison dudit ligand audit récepteur sur lesdites membranes ;
l'incubation desdites membranes en présence dudit ligand et dudit médicament ; et
la détermination du niveau de liaison dudit ligand auxdites membranes, un niveau de liaison plus bas en présence dudit médicament indiquant que ledit composé est un inhibiteur de la liaison du ligand.

32. Procédé pour détecter l'expression d'un polynucléotide codant le récepteur de sérotonine St-B17 selon la revendication 11 dans un échantillon de tissu, comprenant les étapes de
prélèvement dudit échantillon de tissu sur un mammifère ;
mise en contact de l'ARNm dans le cytoplasme dudit échantillon de tissu avec une sonde polynucléotidique marquée présentant une homologie avec ledit polynucléotide codant ledit récepteur de sérotonine St-B 17 ; et
détection de la liaison de ladite sonde avec ledit tissu, une liaison positive indiquant l'expression de la protéine récepteur de sérotonine St-B 17.


### SEQUENCE LISTING

(1) GENERAL INFORMATION:
   (i) APPLICANT: The United States of America as Represented by the Secretary of Health and Human Services
   (ii) TITLE OF INVENTION: THE ST-B17 SEROTONIN RECEPTOR
   (iii) NUMBER OF SEQUENCES: 13
   (iv) CORRESPONDENCE ADDRESS:
      (A) ADDRESSEE: KNOBBE, MARTENS, OLSON AND BEAR
      (B) STREET: 620 NEWPORT CENTER DRIVE 16TH FLOOR
      (C) CITY: NEWPORT BEACH
      (D) STATE: CA
      (E) COUNTRY: USA
      (F) ZIP: 92660
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Altman, Daniel E.
      (B) REGISTRATION NUMBER: 34,115
      (C) REFERENCE/DOCKET NUMBER: NIH047.001VPC
   (ix) TELECOMMUNICATION INFORMATION:
      (A) TELEPHONE: 714-760-0404
      (B) TELEFAX: 714-760-9502
(2) INFORMATION FOR SEQ ID NO:1:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (xi) SEQUENCE DESCRIPTION: SEQ ID NO:1:
   GTCGACCCTK TKSGCCMTCA KCAYRGRTCG CTA 33
(2) INFORMATION FOR SEQ ID NO:2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: YES
   (xi) SEQUENCE DESCRIPTION: SEQ ID NO:2:
   AAGCTTATGA ARAAGGGCAG SCARCAGAGG KYRMA 35
(2) INFORMATION FOR SEQ ID NO:3:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:3:
   AAGCATAGCA GGAAGGCCTT GAAGGCCAGC CTG
(2) INFORMATION FOR SEQ ID NO:4:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:4:
   GGCGAGAAAT ACGCCCTGAA GTTCTCCCGG GAC 33
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:5:
   TTGCCAATAC TACTCTAAGG TGCAGCTTCC 30
(2) INFORMATION FOR SEQ ID NO:6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 1914 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vii) IMMEDIATE SOURCE:
      (B) CLONE: St-B17 without intron
   (ix) FEATURE:
      (A) NAME/KEY: CDS
      (B) LOCATION: 439..1749
   (xi) SEQUENCE DESCRIPTION: SEQ ID NO:7:
   CCAACCCCCA CGCGCGACAC GTGGTGATCT AACGTACTCA CACGCCCACC CTTCTCGAAG      60
   AGACTGCCCC GGCCGGAAGG CGGGAGTTCG GCTCCTGCTC CCACATCCCC AGCTGTGCCC     120
   CTAGCCAGGA ACCCCACCCC CATCTTATGG CATCCCCGGT GGCCCTATTC CATCCCAGGG     180
   CTCTCATCCA GCCCCAAGCT AACTTTCATT GACTCGTCAC ATCAGTACCC CTCCCCAAAC     240
   TTCTTACCCG AGTACTCCAG GTGGCCCTGC GTAGGAGGCA CCCCTACAAC TCCTCCCGAT     300
   CTCTTGAAAT CGCTGCTCGA TGACCTAAGA ACCCCGTTTT GCCAATACTA CTCTAAGGTG     360
   CAGCTTCCTT TCTCCTCCTT TGCCTTCACC CTGTACCTGC AGTCACCATA TCCCGTCTTG     420
   GTCCTCAACC CAGTCCCC ATG GTT CCA GAG CCA GGC CCT GTC AAC AGT AGC      471
                       Met Val Pro Glu Pro Gly Pro Val Asn Ser Ser
                        1               5                  10
   ACC CCA GCC TGG GGT CCC GGG CCA CCG CCT GCT CCG GGG GGC AGC GGC      519
   Thr Pro Ala Trp Gly Pro Gly Pro Pro Pro Ala Pro Gly Gly Ser Gly
               15                  20                  25
   TGG GTG GCT GCC GCG CTG TGC GTG GTC ATC GTG CTG ACA GCA GCC GCC      567
   Trp Val Ala Ala Ala Leu Cys Val Val Ile Val Leu Thr Ala Ala Ala
           30                  35                  40
   AAT TCG CTG CTG ATC GTG CTC ATT TGC ACG CAG CCC GCC GTG CGC AAC      615
   Asn Ser Leu Leu Ile Val Leu Ile Cys Thr Gln Pro Ala Val Arg Asn
       45                  50                  55
   ACG TCT AAC TTC TTT CTG GTG TCG CTC TTC ACG TCG GAC TTG ATG GTG      663
   Thr Ser Asn Phe Phe Leu Val Ser Leu Phe Thr Ser Asp Leu Met Val
   60                  65                  70                  75
   GGG TTG GTG GTG ATG CCC CCA GCC ATG CTG AAC GCG CTG TAT GGG CGC      711
   Gly Leu Val Val Met Pro Pro Ala Met Leu Asn Ala Leu Tyr Gly Arg
                    80                  85                  90
   TGG GTG TTA GCT CGA GGC CTC TGT CTG CTT TGG ACT GCC TTC GAC GTG     759
   Trp Val Leu Ala Arg Gly Leu Cys Leu Leu Trp Thr Ala Phe Asp Val
                95                 100                 105
   ATG TGC TGC AGC GCC TCC ATC CTC AAC CTC TGC CTC ATC AGC CTG GAC     807
   Met Cys Cys Ser Ala Ser Ile Leu Asn Leu Cys Leu Ile Ser Leu Asp
           110                 115                 120
   CGC TAC CTG CTC ATC CTC TCG CCG CTG CGC TAC AAG CTG CGC ATG ACA     855
   Arg Tyr Leu Leu Ile Leu Ser Pro Leu Arg Tyr Lys Leu Arg Met Thr
       125                 130                 135
   GCC CCG CGA GCC CTG GCG CTC ATC CTG GGT GCC TGG AGC CTC GCG GCG     903
   Ala Pro Arg Ala Leu Ala Leu Ile Leu Gly Ala Trp Ser Leu Ala Ala
   140                 145                 150                 155
   CTT GCC TCC TTC CTA CCC CTC TTG CTG GGC TGG CAC GAA CTG GGC AAA     951
   Leu Ala Ser Phe Leu Pro Leu Leu Leu Gly Trp His Glu Leu Gly Lys
                   160                 165                 170
   GCT CGA ACA CCT GCC CCT GGC CAG TGC CGC CTA TTG GCC AGC CTG CCT     999
   Ala Arg Thr Pro Ala Pro Gly Gln Cys Arg Leu Leu Ala Ser Leu Pro
               175                 180                 185
   TTT GTC CTC GTG GCG TCC GGC GTC ACC TTT TTC CTG CCT TCG GGT GCC    1047
   Phe Val Leu Val Ala Ser Gly Val Thr Phe Phe Leu Pro Ser Gly Ala
           190                 195                 200
   ATC TGC TTC ACC TAC TGC AGG ATC CTT CTG GCT GCC CGC AAG CAG GCG    1095
   Ile Cys Phe Thr Tyr Cys Arg Ile Leu Leu Ala Ala Arg Lys Gln Ala
       205                 210                 215
   GTG CAA GTG GCC TCG CTC ACC ACG GGC ACG GCT GGC CAG GCC TTG GAA    1143
   Val Gln Val Ala Ser Leu Thr Thr Gly Thr Ala Gly Gln Ala Leu Glu
   220                 225                 230                 235
   ACC TTG CAG GTG CCC AGG ACA CCA CGC CCA GGG ATG GAG TCC GCT GAC    1191
   Thr Leu Gln Val Pro Arg Thr Pro Arg Pro Gly Met Glu Ser Ala Asp
                   240                 245                 250
   AGT AGG CGT CTG GCC ACC AAG CAT AGC AGG AAG GCC TTG AAG GCC AGC    1239
   Ser Arg Arg Leu Ala Thr Lys His Ser Arg Lys Ala Leu Lys Ala Ser
               255                 260                 265
   CTG ACC CTG GGC ATC CTG CTG GGA ATG TTC TTT GTC ACC TGG CTG CCC    1287
   Leu Thr Leu Gly Ile Leu Leu Gly Met Phe Phe Val Thr Trp Leu Pro
           270                 275                 280
   TTC TTT GTG GCC AAC ATA GCT CAG GCC GTG TGT GAC TGC ATC TCC CCA    1335
   Phe Phe Val Ala Asn Ile Ala Gln Ala Val Cys Asp Cys Ile Ser Pro
       285                 290                 295
   GGC CTC TTC GAT GTC CTC ACA TGG CTG GGG TAC TGT AAT AGC ACC ATG         1383
   Gly Leu Phe Asp Val Leu Thr Trp Leu Gly Tyr Cys Asn Ser Thr Met
   300                 305                 310                 315
   AAC CCT ATC ATC TAC CCG CTC TTT ATG CGG GAC TTC AAG AGG GCC CTG         1431
   Asn Pro Ile Ile Tyr Pro Leu Phe Met Arg Asp Phe Lys Arg Ala Leu
                   320                 325                 330
   GGC AGG TTC CTG CAT GCG TCC ACT GTC CCC CGG AGC ACC GGC CAG CCC         1479
   Gly Arg Phe Leu His Ala Ser Thr Val Pro Arg Ser Thr Gly Gln Pro
               335                 340                 345
   TGC CTC CCC CTC CAT GTG GAC CTC TCA CAG CGG TGC CAG ACC AGG CCT         1527
   Cys Leu Pro Leu His Val Asp Leu Ser Gln Arg Cys Gln Thr Arg Pro
           350                 355                 360
   CAG CTG CAG CAG GTG CTC GCT CTG CCT CTG CCG CCA AAC TCA GAT TCA         1575
   Gln Leu Gln Gln Val Leu Ala Leu Pro Leu Pro Pro Asn Ser Asp Ser
       365                 370                 375
   GAC TCC GCT TCA GGG GGC ACC TCG GGC CTG CAG CTC ACA GCC CAG CTT         1623
   Asp Ser Ala Ser Gly Gly Thr Ser Gly Leu Gln Leu Thr Ala Gln Leu
   380                 385                 390                 395
   CTG CTG CCT GGA GAG GCC ACA CGG GAC CCC CCG CCA CCC ACC AGG GCC         1671
   Leu Leu Pro Gly Glu Ala Thr Arg Asp Pro Pro Pro Pro Thr Arg Ala
                   400                 405                 410
   ACC ACT GTG GTC AAC TTC TTT GTC ACA GAC TCT GTG GAG CCT GAG ATA         1719
   Thr Thr Val Val Asn Phe Phe Val Thr Asp Ser Val Glu Pro Glu Ile
               415                 420                 425
   CGG CCG CAT CCA CTC AGT TCC CCC GTG AAC TGACCAGGTC AAGAGCTGGC           1769
   Arg Pro His Pro Leu Ser Ser Pro Val Asn
           430                 435
   CATTGGAGGC CACATTCCCG GAGCTCTCAG CCCACTCTCC CTGAGACTAG GAGGTGGTAG       1829
   GTCTCCTGAG AGTGTGCTGA ATTGAGGTAT CTCAGCTAGC CCATCTTCTG CTGCAGCTCC       1889
   TTGACTGAGG GGTAGTCAGA CACAT                                             1914
(2) INFORMATION FOR SEQ ID NO:8:
   (i) SEQUENCE CHARACTERISTICS:
      (A) LENGTH: 437 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Val Pro Glu Pro Gly Pro Val Asn Ser Ser Thr Pro Ala Trp Gly
   1               5                   10                  15
   Pro Gly Pro Pro Pro Ala Pro Gly Gly Ser Gly Trp Val Ala Ala Ala
               20                  25                  30
   Leu Cys Val Val Ile Val Leu Thr Ala Ala Ala Asn Ser Leu Leu Ile
           35                  40                  45
   Val Leu Ile Cys Thr Gln Pro Ala Val Arg Asn Thr Ser Asn Phe Phe
       50                  55                  60
   Leu Val Ser Leu Phe Thr Ser Asp Leu Met Val Gly Leu Val Val Met
   65                  70                  75                  80
   Pro Pro Ala Met Leu Asn Ala Leu Tyr Gly Arg Trp Val Leu Ala Arg
                   85                  90                  95
   Gly Leu Cys Leu Leu Trp Thr Ala Phe Asp Val Met Cys Cys Ser Ala
               100                 105                 110
   Ser Ile Leu Asn Leu Cys Leu Ile Ser Leu Asp Arg Tyr Leu Leu Ile
           115                 120                 125
   Leu Ser Pro Leu Arg Tyr Lys Leu Arg Met Thr Ala Pro Arg Ala Leu
       130                 135                 140
   Ala Leu Ile Leu Gly Ala Trp Ser Leu Ala Ala Leu Ala Ser Phe Leu
   145                 150                 155                 160
   Pro Leu Leu Leu Gly Trp His Glu Leu Gly Lys Ala Arg Thr Pro Ala
                   165                 170                 175
   Pro Gly Gln Cys Arg Leu Leu Ala Ser Leu Pro Phe Val Leu Val Ala
               180                 185                 190
   Ser Gly Val Thr Phe Phe Leu Pro Ser Gly Ala Ile Cys Phe Thr Tyr
           195                 200                 205
   Cys Arg Ile Leu Leu Ala Ala Arg Lys Gln Ala Val Gln Val Ala Ser
       210                 215                 220
   Leu Thr Thr Gly Thr Ala Gly Gln Ala Leu Glu Thr Leu Gln Val Pro
   225                 230                 235                 240
   Arg Thr Pro Arg Pro Gly Met Glu Ser Ala Asp Ser Arg Arg Leu Ala
                   245                 250                 255
   Thr Lys His Ser Arg Lys Ala Leu Lys Ala Ser Leu Thr Leu Gly Ile
               260                 265                 270
   Leu Leu Gly Met Phe Phe Val Thr Trp Leu Pro Phe Phe Val Ala Asn
           275                 280                 285
   Ile Ala Gln Ala Val Cys Asp Cys Ile Ser Pro Gly Leu Phe Asp Val
       290                 295                 300
   Leu Thr Trp Leu Gly Tyr Cys Asn Ser Thr Met Asn Pro Ile Ile Tyr
   305                 310                 315                 320
   Pro Leu Phe Met Arg Asp Phe Lys Arg Ala Leu Gly Arg Phe Leu His
                   325                 330                 335
   Ala Ser Thr Val Pro Arg Ser Thr Gly Gln Pro Cys Leu Pro Leu His
               340                 345                 350
   Val Asp Leu Ser Gln Arg Cys Gln Thr Arg Pro Gln Leu Gln Gln Val
           355                 360                 365
   Leu Ala Leu Pro Leu Pro Pro Asn Ser Asp Ser Asp Ser Ala Ser Gly
       370                 375                 380
   Gly Thr Ser Gly Leu Gln Leu Thr Ala Gln Leu Leu Leu Pro Gly Glu
   385                 390                 395                 400
   Ala Thr Arg Asp Pro Pro Pro Pro Thr Arg Ala Thr Thr Val Val Asn
                   405                 410                 415
   Phe Phe Val Thr Asp Ser Val Glu Pro Glu Ile Arg Pro His Pro Leu
               420                 425                 430
   Ser Ser Pro Val Asn
           435
(2) INFORMATION FOR SEQ ID NO:9:
   (i) SEQUENCE CHARACTERISTICS:
      (A) LENGTH: 2108 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vii) IMMEDIATE SOURCE:
      (B) CLONE: St-B17 cDNA clone
   (ix) FEATURE:
      (A) NAME/KEY: CDS
      (B) LOCATION: 439..1311
   (ix) FEATURE:
      (A) NAME/KEY: intron
      (B) LOCATION: 1312..1505
   (ix) FEATURE:
      (A) NAME/KEY: CDS
      (B) LOCATION: 1506..1943
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 291 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Val Pro Glu Pro Gly Pro Val Asn Ser Ser Thr Pro Ala Trp Gly
    1               5                  10                  15
   Pro Gly Pro Pro Pro Ala Pro Gly Gly Ser Gly Trp Val Ala Ala Ala
               20                  25                  30
   Leu Cys Val Val Ile Val Leu Thr Ala Ala Ala Asn Ser Leu Leu Ile
           35                  40                  45
   Val Leu Ile Cys Thr Gln Pro Ala Val Arg Asn Thr Ser Asn Phe Phe
   50 55 60
   Leu Val Ser Leu Phe Thr Ser Asp Leu Met Val Gly Leu Val Val Met
   65 70 75 80
   Pro Pro Ala Met Leu Asn Ala Leu Tyr Gly Arg Trp Val Leu Ala Arg
   85 90 95
   Gly Leu Cys Leu Leu Trp Thr Ala Phe Asp Val Met Cys Cys Ser Ala
   100 105 110
   Ser Ile Leu Asn Leu Cys Leu Ile Ser Leu Asp Arg Tyr Leu Leu Ile
   115 120 125
   Leu Ser Pro Leu Arg Tyr Lys Leu Arg Met Thr Ala Pro Arg Ala Leu
   130 135 140
   Ala Leu Ile Leu Gly Ala Trp Ser Leu Ala Ala Leu Ala Ser Phe Leu
   145 150 155 160
   Pro Leu Leu Leu Gly Trp His Glu Leu Gly Lys Ala Arg Thr Pro Ala
   165 170 175
   Pro Gly Gln Cys Arg Leu Leu Ala Ser Leu Pro Phe Val Leu Val Ala
   180 185 190
   Ser Gly Val Thr Phe Phe Leu Pro Ser Gly Ala Ile Cys Phe Thr Tyr
   195 200 205
   Cys Arg Ile Leu Leu Ala Ala Arg Lys Gln Ala Val Gln Val Ala Ser
   210 215 220
   Leu Thr Thr Gly Thr Ala Gly Gln Ala Leu Glu Thr Leu Gln Val Pro
   225 230 235 240
   Arg Thr Pro Arg Pro Gly Met Glu Ser Ala Asp Ser Arg Arg Leu Ala
   245 250 255
   Thr Lys His Ser Arg Lys Ala Leu Lys Ala Ser Leu Thr Leu Gly Ile
   260 265 270
   Leu Leu Gly Met Phe Phe Val Thr Trp Leu Pro Phe Phe Val Ala Asn
   275 280 285
   Ile Ala Gln
   290
(2) INFORMATION FOR SEQ ID NO:11:
   (i) SEQUENCE CHARACTERISTICS:
      (A) LENGTH: 146 amino acids
      (B) TYPE: amino acid
      (C) TOPOLOGY: linear
   (ii) MOLECULE TYPE: protein
   (xi) SEQUENCE DESCRIPTION: SEQ ID NO:11:
   Ala Val Cys Asp Cys Ile Ser Pro Gly Leu Phe Asp Val Leu Thr Trp
    1               5                  10                  15
   Leu Gly Tyr Cys Asn Ser Thr Met Asn Pro Ile Ile Tyr Pro Leu Phe
               20                  25                  30
   Met Arg Asp Phe Lys Arg Ala Leu Gly Arg Phe Leu His Ala Ser Thr
           35                  40                  45
   Val Pro Arg Ser Thr Gly Gln Pro Cys Leu Pro Leu His Val Asp Leu
       50                  55                  60
   Ser Gln Arg Cys Gln Thr Arg Pro Gln Leu Gln Gln Val Leu Ala Leu
   65                  70                  75                  80
   Pro Leu Pro Pro Asn Ser Asp Ser Asp Ser Ala Ser Gly Gly Thr Ser
                   85                  90                  95
   Gly Leu Gln Leu Thr Ala Gln Leu Leu Leu Pro Gly Glu Ala Thr Arg
               100                 105                 110
   Asp Pro Pro Pro Pro Thr Arg Ala Thr Thr Val Val Asn Phe Phe Val
           115                 120                 125
   Thr Asp Ser Val Glu Pro Glu Ile Arg Pro His Pro Leu Ser Ser Pro
       130                 135                 140
   Val Asn
   145
(2) INFORMATION FOR SEQ ID NO:12:
   (i) SEQUENCE CHARACTERISTICS:
      (A) LENGTH: 1647 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vii) IMMEDIATE SOURCE:
      (B) CLONE: Human STB-17
   (ix) FEATURE:
      (A) NAME/KEY: CDS
      (B) LOCATION: 135..1454
   (xi) SEQUENCE DESCRIPTION: SEQ ID NO:12:
   ACTCACCTCC CCCGGGGGGC GTGGTGAGTC GCGGTCTGTT CTCACGACGG TCCCCGTCCA      60
   GCCTGCGGTT CGCCGGGGCC CTCATCTGCT TTCCCGCCAC CCTATCACTC CCTTGCCGTC     120
   CACCCTCGGT CCTC ATG GTC CCA GAG CCG GGC CCA ACC GCC AAT AGC ACC       170
                   Met Val Pro Glu Pro Gly Pro Thr Ala Asn Ser Thr
                    1               5                  10
   CCG GCC TGG GGG GCA GGC GCC CGT CGN NNG GGG GGC AGC GGC TGG GTG       218
   Pro Ala Trp Gly Ala Gly Ala Arg Xaa Xaa Gly Gly Ser Gly Trp Val
           15                  20                  25
   GCG GCC GGC CTG TGC GTG GTC ATC GCG CTG ACG GCG GCG GCC AAC TCG       266
   Ala Ala Gly Leu Cys Val Val Ile Ala Leu Thr Ala Ala Ala Asn Ser
       30                  35                  40
   CTG CTG ATC GCG CTC ATC TGC ACT CAG CCC GCG CTG CGC AAC ACG TCC       314
   Leu Leu Ile Ala Leu Ile Cys Thr Gln Pro Ala Leu Arg Asn Thr Ser
   45                  50                  55                  60
   AAC TTC TTC CTG GTG TCG CTC TTC ACG TCT GAC CTG ATG GTC GGG CTG       362
   Asn Phe Phe Leu Val Ser Leu Phe Thr Ser Asp Leu Met Val Gly Leu
                   65                  70                  75
   GTG GTG ATG CCG CCG GCC ATG CTG AAC GCG CTG TAC GGG CGC TGG GTG       410
   Val Val Met Pro Pro Ala Met Leu Asn Ala Leu Tyr Gly Arg Trp Val
               80                  85                  90
   CTG GCG CGC GGC CTC TGC CTG CTC TGG ACC GCC TTC GAC GTG ATG TGC       458
   Leu Ala Arg Gly Leu Cys Leu Leu Trp Thr Ala Phe Asp Val Met Cys
           95                 100                 105
   TGC AGC GCC TCC ATC CTC AAC CTC TGC CTC ATC AGC CTG GAC CGC TAC       506
   Cys Ser Ala Ser Ile Leu Asn Leu Cys Leu Ile Ser Leu Asp Arg Tyr
       110                 115                 120
   CTG CTC ATC CTC TCG CCG CTG CGC TAC AAG CTG CGC ATG ACG CCC CTG       554
   Leu Leu Ile Leu Ser Pro Leu Arg Tyr Lys Leu Arg Met Thr Pro Leu
   125                 130                 135                 140
   CGT GCC CTG GCC CTA GTC CTG GGC GGC TGG AGC CTC GCC GCT CTC GCC       602
   Arg Ala Leu Ala Leu Val Leu Gly Gly Trp Ser Leu Ala Ala Leu Ala
                   145                 150                 155
   TCC TTC CTG CCC CTG CTG CTG GGC TGG CAC GAG CTG GGC CAC GCA CGG       650
   Ser Phe Leu Pro Leu Leu Leu Gly Trp His Glu Leu Gly His Ala Arg
               160                 165                 170
   CCA CCC GTC CCT GGC CAG TGC CGC CTG CTG GCC AGC CTG CCT TTT GTC       698
   Pro Pro Val Pro Gly Gln Cys Arg Leu Leu Ala Ser Leu Pro Phe Val
           175                 180                 185
   CTT GTG GCG TCG GGC CTC ACC TTC TTC CTG CCC TCG GGT GCC ATA TGC        746
   Leu Val Ala Ser Gly Leu Thr Phe Phe Leu Pro Ser Gly Ala Ile Cys
       190                 195                 200
   TTC ACC TAC TGC AGG ATC CTG CTA GCT GCC CGC AAG CAG GCC GTG CAG        794
   Phe Thr Tyr Cys Arg Ile Leu Leu Ala Ala Arg Lys Gln Ala Val Gln
   205                 210                 215                 220
   GTG GCC TCC CTC ACC ACC GGC ATG GCC AGT CAG GCC TCG GAG ACG CTG        842
   Val Ala Ser Leu Thr Thr Gly Met Ala Ser Gln Ala Ser Glu Thr Leu
                   225                 230                 235
   CAG GTA CCC AGG AGC CCA GCG GCA GGG GTG GAG TCT GCT GAC AGC AGG        890
   Gln Val Pro Arg Ser Pro Ala Ala Gly Val Glu Ser Ala Asp Ser Arg
               240                 245                 250
   CGT CTA GCA ACG AAG AGC AGC AGG AAG GGC CTG AAG GCC AGC ATG ACG        938
   Arg Leu Ala Thr Lys Ser Ser Arg Lys Gly Leu Lys Ala Ser Met Thr
           255                 260                 265
   CTG GGC ATC CTG CTG GGC ATG TTC TTT GTG ACC TGG TTG CCC TTC TTT        986
   Leu Gly Ile Leu Leu Gly Met Phe Phe Val Thr Trp Leu Pro Phe Phe
       270                 275                 280
   GTG GCC AAC ATA GTC CAG GCC GTG TGC GAC TGC ATC TCC CCA GGC CTC       1034
   Val Ala Asn Ile Val Gln Ala Val Cys Asp Cys Ile Ser Pro Gly Leu
   285                 290                 295                 300
   TTC GAT GTC CTC ACA TGG CTG GGT TAC TGT AAC AGC ACC ATG AAC CCC       1082
   Phe Asp Val Leu Thr Trp Leu Gly Tyr Cys Asn Ser Thr Met Asn Pro
                   305                 310                 315
   ATC ATC TAC CCA CTC TTC ATG CTG GAC TTC AAG CGG GCG CTG GGC AGG       1130
   Ile Ile Tyr Pro Leu Phe Met Leu Asp Phe Lys Arg Ala Leu Gly Arg
               320                 325                 330
   TTC CTG CCA TGT CCA CGC TGT CCC CGG GAG CCC AGG CCA GCC TGG CCT       1178
   Phe Leu Pro Cys Pro Arg Cys Pro Arg Glu Pro Arg Pro Ala Trp Pro
           335                 340                 345
   CGC CAT CAC TGC GCA CCT CTC ACA GCG GCC CCC GGC CCG GCC TTA GCC       1226
   Arg His His Cys Ala Pro Leu Thr Ala Ala Pro Gly Pro Ala Leu Ala
       350                 355                 360
   TAC AGC AGG TGC TGC CGC TGC CCC TGC CGC CGG ACT CAG ATT CGG ACT       1274
   Tyr Ser Arg Cys Cys Arg Cys Pro Cys Arg Arg Thr Gln Ile Arg Thr
   365                 370                 375                 380
   CAG ACG CAG GCT CAG GCG GCT CCT CGG GCG TGC GGC TCA CGG CCC AGC       1322
   Gln Thr Gln Ala Gln Ala Ala Pro Arg Ala Cys Gly Ser Arg Pro Ser
                   385                 390                 395
   TGC TGC TTC CTG GCG AGG CCA CCC AGG ACC CCC CGC TGC CCA CCA GGG     1370
   Cys Cys Phe Leu Ala Arg Pro Pro Arg Thr Pro Arg Cys Pro Pro Gly
               400                 405                 410
   CCG CTG CCG CCG TCA ATT TCT TCA ACA TCG SAC CCC GCG GAG CCC GAG     1418
   Pro Leu Pro Pro Ser Ile Ser Ser Thr Ser Xaa Pro Ala Glu Pro Glu
           415                 420                 425
   CTG CGG CCG CAT CCA CTT GGC ATC CCC ACG AAC TGACCCGGCT TGGGGCTGGC   1471
   Leu Arg Pro His Pro Leu Gly Ile Pro Thr Asn
       430                 435                 440
   CAATGGGGAG CTGGATTGAG CAGAACCCAG ACCCTGAGTC CTTGGGCCAG CTCTTGGCTA   1531
   AGACCAGGAG GCTGCAAGTC TCCTAGAAGC CCTCTGAGCT CCAGAGGGGT GCGGCAGAGC   1591
   TGACCCCCTG CTGCCATCTC CAGGCCCCTT ACCTGCAGGG ATCATAGCTG ACTAGA       1647
(2) INFORMATION FOR SEQ ID NO:13:
   (i) SEQUENCE CHARACTERISTICS:
      (A) LENGTH: 439 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Val Pro Glu Pro Gly Pro Thr Ala Asn Ser Thr Pro Ala Trp Gly
   1               5                   10                  15
   Ala Gly Ala Arg Xaa Xaa Gly Gly Ser Gly Trp Val Ala Ala Gly Leu
               20                  25                  30
   Cys Val Val Ile Ala Leu Thr Ala Ala Ala Asn Ser Leu Leu Ile Ala
           35                  40                  45
   Leu Ile Cys Thr Gln Pro Ala Leu Arg Asn Thr Ser Asn Phe Phe Leu
       50                  55                  60
   Val Ser Leu Phe Thr Ser Asp Leu Met Val Gly Leu Val Val Met Pro
   65                  70                  75                  80
   Pro Ala Met Leu Asn Ala Leu Tyr Gly Arg Trp Val Leu Ala Arg Gly
                   85                  90                  95
   Leu Cys Leu Leu Trp Thr Ala Phe Asp Val Met Cys Cys Ser Ala Ser
               100                 105                 110
   Ile Leu Asn Leu Cys Leu Ile Ser Leu Asp Arg Tyr Leu Leu Ile Leu
           115                 120                 125
   Ser Pro Leu Arg Tyr Lys Leu Arg Met Thr Pro Leu Arg Ala Leu Ala
   130 135 140
   Leu Val Leu Gly Gly Trp Ser Leu Ala Ala Leu Ala Ser Phe Leu Pro
   145 150 155 160
   Leu Leu Leu Gly Trp His Glu Leu Gly His Ala Arg Pro Pro Val Pro
   165 170 175
   Gly Gln Cys Arg Leu Leu Ala Ser Leu Pro Phe Val Leu Val Ala Ser
   180 185 190
   Gly Leu Thr Phe Phe Leu Pro Ser Gly Ala Ile Cys Phe Thr Tyr Cys
   195 200 205
   Arg Ile Leu Leu Ala Ala Arg Lys Gln Ala Val Gln Val Ala Ser Leu
   210 215 220
   Thr Thr Gly Met Ala Ser Gln Ala Ser Glu Thr Leu Gln Val Pro Arg
   225 230 235 240
   Ser Pro Ala Ala Gly Val Glu Ser Ala Asp Ser Arg Arg Leu Ala Thr
   245 250 255
   Lys Ser Ser Arg Lys Gly Leu Lys Ala Ser Met Thr Leu Gly Ile Leu
   260 265 270
   Leu Gly Met Phe Phe Val Thr Trp Leu Pro Phe Phe Val Ala Asn Ile
   275 280 285
   Val Gln Ala Val Cys Asp Cys Ile Ser Pro Gly Leu Phe Asp Val Leu
   290 295 300
   Thr Trp Leu Gly Tyr Cys Asn Ser Thr Met Asn Pro Ile Ile Tyr Pro
   305 310 315 320
   Leu Phe Met Leu Asp Phe Lys Arg Ala Leu Gly Arg Phe Leu Pro Cys
   325 330 335
   Pro Arg Cys Pro Arg Glu Pro Arg Pro Ala Trp Pro Arg His His Cys
   340 345 350
   Ala Pro Leu Thr Ala Ala Pro Gly Pro Ala Leu Ala Tyr Ser Arg Cys
   355 360 365
   Cys Arg Cys Pro Cys Arg Arg Thr Gln Ile Arg Thr Gln Thr Gln Ala
   370 375 380
   Gln Ala Ala Pro Arg Ala Cys Gly Ser Arg Pro Ser Cys Cys Phe Leu
   385 390 395 400
   Ala Arg Pro Pro Arg Thr Pro Arg Cys Pro Pro Gly Pro Leu Pro Pro
   405 410 415
   Ser Ile Ser Ser Thr Ser Xaa Pro Ala Glu Pro Glu Leu Arg Pro His
   420 425 430
   Pro Leu Gly Ile Pro Thr Asn
   435